# EUROPÄISCHE PATENTSCHRIFT

(11) **EP 3 219 274 B1**
(45) Veröffentlichungstag und Bekanntmachung des Hinweises auf die Patenterteilung: **08.05.2019**
(21) Anmeldenummer: 17161068.6
(22) Anmeldetag: 15.03.2017
(51) Int. Cl.: A61B 17/88, A61B 17/56, B01F 13/00, B05C 17/01, B01F 5/00, B01F 3/10

(54) **LAGERUNGS- UND MISCHSYSTEM FÜR PASTENFÖRMIGE ZEMENTKOMPONENTEN**
STORAGE AND MIXING SYSTEM FOR PASTY CEMENT COMPONENTS
SYSTÈME DE STOCKAGE ET DE MÉLANGE POUR COMPOSANTS DE CIMENT PÂTEUX

(30) Priorität: 17.03.2016 DE 102016104950
(43) Veröffentlichungstag der Anmeldung: 20.09.2017
(73) Patentinhaber: Heraeus Medical GmbH, 61273 Wehrheim (DE)
(72) Erfinder: Vogt, Sebastian Dr., 99092 Erfurt (DE); Kluge, Thomas Dr., 56179 Vallendar (DE)
(74) Vertreter: Schultheiss & Sterzel Patentanwälte PartG mbB

(56) Entgegenhaltungen:
- EP-A2- 0 119 847
- WO-A1-2011/137971
- DE-A1- 2 521 392
- DE-A1-102013 107 955
- DE-U1-202014 102 416

## Beschreibung

Die Erfindung betrifft eine Lagerungs- und Mischsystem für pastenförmige Zweikomponenten-Polymethylmethacrylat-Knochenzemente, das Lagerungs- und Mischsystem aufweisend eine röhrenförmige Kartusche mit einem zylindrischen Innenraum.

Die Erfindung betrifft auch ein Verfahren zur Vermischung von pastenförmigen Zementkomponenten eines Zementteigs, insbesondere eines pastenförmigen Polymethylmethacrylat-Knochenzements, mit einem solchen Lagerungs- und Mischsystem.

Gegenstand der Erfindung ist somit ein einfaches, kostengünstig herzustellendes Lagerungs- und Mischsystem für pastenförmige Zweikomponenten-Polymethylmethacrylat-Knochenzemente, mit dem die hochviskosen, pastenförmigen Zementkomponenten des Polymethylmethacrylat-Knochenzements auch mit manuell bedienbaren Austragsvorrichtungen gemischt und ausgetragen werden können.

Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Diese Polymethylmethacrylat-Knochenzemente werden nach Vermischung des Zementpulvers mit der flüssigen Monomerkomponente im noch nicht ausgehärteten, pastenförmigen Zustand als Zementteig appliziert. Bei Verwendung von Mischsystemen befindet sich der Zementteig im Fall von Pulver-Flüssigkeitszementen in einer Kartusche. Aus dieser Kartusche wird der Zementteig durch Bewegung eines Austragskolbens heraus gedrückt. Die Austragskolben haben üblicherweise einen Durchmesser zwischen 30 mm bis 40 mm und damit an der Außenseite, an welcher der Stößel der Austragsvorrichtung beim Auspressvorgang angreift, eine Fläche von 7,0 cm² bis 12,5 cm². Die Bewegung des Austragskolbens wird durch manuell bedienbare, mechanische Austragsvorrichtungen bewirkt, die auch als Applikatoren bezeichnet werden. Diese Austragsvorrichtungen beziehungsweise Applikatoren haben normalerweise eine Auspresskraft im Bereich von ungefähr 1,5 kN bis 3,5 N.

Eine neuere Entwicklung stellen pastenförmige Zweikomponenten-Knochenzemente dar, wie sie beispielsweise aus der DE 10 2007 050 762 B3, der DE 10 2008 030 312 A1 und der DE 10 2007 052 116 B4 bekannt sind. Bei diesen Zweikomponenten-Knochenzementen werden zwei pastenförmige Zementkomponenten in zwei separaten Kartuschen mit zwei separaten Austragskolben aufbewahrt. Bei der Applikation werden beide Pasten durch Bewegung der Austragkolben aus den Kartuschen in einen statischen Mischer gepresst und nach erfolgter Vermischung durch ein Austragsrohr ausgetragen. Bei geeigneter Zusammensetzung der pastenförmigen Zementkomponenten wird nach der Vermischung der beiden Zementkomponenten sofort ein klebfreier, applikationsbereiter Zementteig erhalten. Wartezeiten bis zum Erreichen der Klebfreiheit des Zementteigs, die bei bisherigen konventionellen Polymethylmethacrylat-Knochenzementen immer zwangsläufig auftreten, entfallen dadurch. Wertvolle OP-Zeit kann dadurch gespart werden. WO2011/137971 offenbart ein Mischsystem für Knochenzemente, das aus zwei Kartuschen mit verbundenen Förderkolben und einem Austragsrohr mit einem Mischer besteht, wobei die Zementkomponenten aus den Kartuschen in das Rohr gedrückt und dort gemischt werden.

Eigene Versuche im Rahmen der vorliegenden Erfindung zeigten, dass während des Auspressvorgangs der Kartuschen auf Grund der hohen Viskosität der pastenförmigen Zementkomponenten ein sehr großer Druckabfall am statischen Mischer im Austragsrohr stattfindet. Eigene Versuche zeigten weiterhin, dass bei einem konischen Austragsrohr und einer Gesamtlänge von ca. 17 cm und mit einem Innendurchmesser von 11 mm am Kartuschenkopf und unter Verwendung von zehn statischen Mischelementen eine Auspresskraft größer 7 kN notwendig ist, um die hochviskosen Zementpasten in einer für den medizinischen Anwender akzeptablen Austragsgeschwindigkeit auszupressen.

Bei der Applikation der bisherigen, konventionellen PMMA-Knochenzemente, die aus einer flüssigen Monomerkomponente und einer separat dazu gelagerten Zementpulverkomponente bestehen, wird nach der Vermischung der beiden Zementkomponenten in Zementiersystemen beziehungsweise Vakuumzementiersystemen der gebildete Zementteig mit Hilfe von manuell bedienbaren Austragsvorrichtungen ausgepresst. Diese einfachen mechanischen Austragsvorrichtungen nutzen insbesondere Klemmstangen zum Auspressen, die durch einen manuell zu betätigenden Kipphebel angetrieben werden. Die manuell angetriebenen Austragsvorrichtungen sind seit Jahrzehnten weltweit erprobt und stellen bisher den Stand der Technik dar. Vorteilhaft an diesen Austragsvorrichtungen ist, dass der medizinische Anwender über die aufzubringende Handkraft ein Gefühl für den Eindringwiderstand des Knochenzementteigs in die Knochenstrukturen (Spongiosa) hat.

Bei hochviskosen pastenförmigen Zementkomponenten unter Verwendung von Kartuschen, bei denen die Austragskolben an den äußeren Kolbenseiten, an denen die Stößel der Austragsvorrichtungen angreifen, eine gesamte Fläche im Bereich von 7,0 cm² bis 12,5 cm² haben, sind diese Vorrichtungen nicht oder nur mit einem sehr großen Kraftaufwand manuell zu bedienen. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A; DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1; US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Die EP 0 119 847 A2 und die DE 25 21 392 A1 offenbaren Kartuschen mit einer axialen Trennwand, die mit Scheiden aufgetrennt wird, während ein Austragskolben in der Kartusche vorgetrieben wird. Im aus EP 0 119 847 bekannten System teilt die Trennwand den Innenraum der Kartusche in zwei Hohlräume, in denen z.B. die zu mischenden viskosen Komponenten eines Epoxidklebers enthalten sind, wobei in den Hohlräumen zwei verschiebbare Austragskolben angeordnet sind, und die Scheiden und eine Umlenkeinrichtung derart mit den Austragskolben verbunden sind, dass eine geneigte Ablenkfläche der Umlenkeinrichtung die aufgetrennte Trennwand seitlich in Richtung der Innenwand der Kartusche drückt.

Die US 8,544,683 B2 offenbart ein Kartuschensystem, das zur Beimischen einer geringen Menge zu einer Hauptkomponente geeignet ist. Bei dem Kartuschensystem ist neben einer Kartusche eine zweite kleinere Kartusche angeordnet, wobei bei einem Vortrieb eines Austragskolbens in der größeren Kartusche auch ein Austragskolben in der kleineren Kartusche über ein gemeinsames Verbindungselement angetrieben wird. Das System ist zum Mischen der zähen pastösen Zementkomponenten vom PMMA-Knochenzement jedoch nicht geeignet.

Ein koaxiales Kartuschensystem, das ein spezielles Kolbensystem enthält, wird im Patent EP1 392 450 B1 beschrieben. Das Kartuschensystem findet Anwendung in der Baustoffchemie für die Lagerung und Vermischung von pastenförmigen Zweikomponentendichtungsmassen. Das dort offenbarte Kolbensystem hat einen zylinderförmigen Austragskolben für die zentrale Kartusche und einen ringförmigen Austragskolben für die zweite, koaxial angeordnete Kartusche. Beide Austragskolben werden hinter den Dichtungsflächen über ein Auflageelement angetrieben, das auf der Rückseite mehrere Anlageflächen für den Stößel der Austragsvorrichtung besitzt. Das Auflageelement enthält bogenförmige Schneiden. Bei axialer Einwirkung des Stößels einer Auspressvorrichtung werden beide Kolben vorwärts in Richtung Kartuschenkopf bewegt.

Dabei werden die in den koaxialen Kartuschen enthaltenen pastenförmigen Komponenten in Richtung Kartuschenkopf gedrückt. Gleichzeitig zerteilen zwei Schneiden die Wand der inneren koaxialen Kartusche in zwei Teile. Nachteilig ist an diesem System, dass zwangsläufig gleichzeitig zwei Schneidprozesse ablaufen. Das bedeutet, dass für beide Schneidprozesse Energie aufgebracht werden muss, die dadurch nicht zum eigentlichen Vortrieb der beiden pastösen Komponenten zur Verfügung steht. Die Vermischung von pastenförmigen Zementkomponenten für PMMA-Knochenzemente benötigt aufgrund der im Austragsrohr angeordneten statischen Mischer und der hohen Viskosität der Zementkomponenten sehr viel Vortriebsenergie, der bei größeren Kartuschen nicht mehr manuell, gefahrlos und nicht mit herkömmlichen Austragsvorrichtungen aufzubringen ist. Ein Verlust an Vortriebsenergie durch zwei gleichzeitig stattfindende Schneidprozesse kann daher insbesondere bei hoch viskosen pastenförmigen Komponenten problematisch sein. Zudem sind Koaxialkartuschen nicht ohne weiteres mit Zementkomponenten eines PMMA-Knochenzements zu befüllen. Insbesondere wenn nur geringe Mengen des PMMA-Knochenzements enthalten sein sollen, werden die freien Querschnitte der äußeren Koaxialkartusche so klein, dass sie nicht mit herkömmlichen Verfahren zu befüllen ist.

Die Aufgabe der Erfindung besteht also darin, die Nachteile des Stands der Technik zu überwinden. Insbesondere soll ein einfaches, kostengünstig zu fertigendes Lagerungs- und Mischsystem für pastenförmige Zweikomponenten-Polymethylmethacrylat-Knochenzemente und ein Verfahren zur Herstellung eines Zementteigs mit einem Lagerungs- und Mischsystem bereitgestellt werden, wobei das Lagerungs- und Mischsystem als einmalig zu verwendendes ready-to-use-System in einfachster Weise mit einer minimalen Anzahl von Montageschritten innerhalb weniger Sekunden einsatzbereit machbar sein soll und nach Verbindung mit manuell anzutreibenden medizinischen Austragsvorrichtungen beziehungsweise Applikatoren sofort nach Beginn der manueller Betätigung der Austragsvorrichtung einen homogen gemischten Zementteig erzeugt und an der Austragsöffnung eines Austragsrohrs austrägt. Die in den OPs bisher für die konventionellen Polymethylmethacrylat-Knochenzemente genutzten manuell zu bedienenden Austragsvorrichtungen mit jeweils einer Schubstange und gegebenenfalls einem Teller sollen für den Austrag des Zweikomponenten-Polymethylmethacrylat-Knochenzements beziehungsweise des Zementteigs mit dem zu entwickelnden Lagerungs- und Mischsystem genutzt werden können. Dadurch soll die Beschaffung von speziellen Austragsvorrichtungen für den Austrag von pastenförmigen Zweikomponenten-Polymethylmethacrylat-Knochenzementen vermieden werden.

Bevorzugt soll das zu entwickelnde Lagerungs- und Mischsystem keine zwei miteinander verbundenen synchron vorzutreibenden Schubstangen beziehungsweise Stößel erfordern, damit die gesamte Vorrichtung nicht wesentlich länger und größer wird als die bisher für die konventionellen Pulver-Flüssigkeits-Polymethylmethacrylat-Knochenzemente üblichen Mischsysteme und Vakuummischsysteme. Es soll eine einfache Lösung gefunden werden, die es erlaubt, möglichst mit nur einer Schubstange beziehungsweise nur einem Stößel und gegebenenfalls einem daran befestigten Teller zwei pastenförmige Zementkomponenten synchron und manuell aus der Vorrichtung auszutreiben. Die pastenförmigen Zementkomponenten des Knochenzements sollen getrennt innerhalb des Lagerungs- und Mischsystems sicher gelagert werden können. Zur Anwendung sollen beide pastenförmigen Zementkomponenten sicher zusammengeführt werden können. Das Lagerungs- und Mischsystem soll auch ein geringes Volumen des homogen gemischten Zementteigs von ca. 50 ml beziehungsweise maximal 70 ml austragen können, ohne dass größere Restmengen (mehr als 15 ml) in dem System verbleiben und aufwendig entsorgt werden müssen. Größere Volumina des Zementteigs werden nicht angestrebt. Die genannten geringen Mengen sind nämlich für viele Anwendungen ausreichend, wie beispielsweise Operationen (OPs) im Bereich des Knies.

Der Übergang von der Kartusche zum Austragsrohr soll konstruktiv möglichst so gestaltet sein, dass der Strömungswiderstand der pastenförmigen Zementkomponenten beim Auspressen möglichst niedrig ist. Als Zementkomponenten müssen pastenförmige Zementkomponenten verwendet werden, die unmittelbar nach dem Auspressen anwendbar sind, bei denen also keine Zeit zum Anquellen des PMMA-Knochenzements notwendig ist. Die Vorrichtung soll so beschaffen sein, dass eine Verwechselung der relevanten Montageschritte durch den Anwender konstruktiv soweit wie möglich ausgeschlossen ist und das Lagerungs- und Mischsystem auch von weitgehend ungeschultem Personal durchführbar ist. Weiterhin soll ein Verfahren zum Vermischen der pastenförmigen Zementkomponenten und zum Austragen des homogen gemischten Zementteigs bereitgestellt werden.

Die Aufgaben der Erfindung werden gelöst durch ein Lagerungs- und Mischsystem für pastenförmige Zweikomponenten-Polymethylmethacrylat-Knochenzemente, das Lagerungs- und Mischsystem aufweisend
eine röhrenförmige Kartusche mit einem zylindrischen Innenraum,
einen Kartuschenkopf, der ein Ende der röhrenförmigen Kartusche an der Vorderseite verschließt,
eine axial in dem zylindrischen Innenraum der Kartusche angeordnete Trennwand, wobei die Trennwand den durch den Kartuschenkopf begrenzten zylindrischen Innenraum der Kartusche in zwei räumlich voneinander getrennte Hohlräume teilt, wobei in dem ersten Hohlraum eine erste pastenförmige Zementkomponente enthalten ist und in dem separaten zweiten Hohlraum eine zweite pastenförmige Zementkomponente enthalten ist,
zwei axial in beiden Hohlräumen der Kartusche verschiebbar angeordnete Austragskolben, wobei die Austragskolben die beiden Hohlräume auf der dem Kartuschenkopf gegenüberliegenden Rückseite der Hohlräume verschließen,
eine Umlenkeinrichtung zum Umformen der Trennwand, wobei die Umlenkeinrichtung in der Kartusche in axial bewegbar ist und vom Kartuschenkopf aus gesehen hinter den Austragskolben angeordnet ist oder anzuordnen ist, wobei die Umlenkeinrichtung eine gegen die Achse der Kartusche geneigte Ablenkfläche aufweist, die die Trennwand seitlich in Richtung der Innenwand der Kartusche drückt, wenn die Umlenkeinrichtung in die Kartusche hinein gedrückt wird, wobei
die Trennwand mit der Innenwand der Kartusche über eine Sollbruchstelle verbunden ist oder die Trennwand mit der Innenwand der Kartusche lösbar verbunden ist, so dass sich die Trennwand von der Innenwand der Kartusche löst, wenn die Trennwand durch die Bewegung der Umlenkeinrichtung in die Kartusche hinein verformt wird, wobei
ein rückseitiges Ende der Trennwand, das vom Kartuschenkopf aus gesehen hinter den Austragskolben angeordnet ist, an der Kartusche zu befestigen ist oder befestigt ist, so dass sich das rückseitige Ende der Trennwand bei einer Bewegung der Umlenkeinrichtung in die Kartusche hinein nicht von der Kartusche löst.

Die Umlenkeinrichtung wird von der Rückseite aus in die Kartusche hinein gedrückt.

Es ist erfindungsgemäß bevorzugt, wenn die Trennwand nicht fest mit der Kartusche verbunden ist und/oder wenn die Trennwand als separates Teil in der Kartusche angeordnet ist. Dann lässt sich die Trennwand leichter und kontrollierter von der Innenwand der Kartusche lösen und es wird weniger Energie zum Vortreiben der Umlenkeinrichtung benötigt, da die Trennwand nicht mechanisch von der Innenwand der Kartusche abgetrennt werden muss.

Bei erfindungsgemäßen Lagerungs- und Mischsystemen kann vorgesehen sein, dass ein rückseitiges Ende der Trennwand, das vom Kartuschenkopf aus gesehen hinter den Austragskolben angeordnet ist, an der Kartusche zu befestigen ist oder befestigt ist, so dass sich das rückseitige Ende der Trennwand bei einer Bewegung der Umlenkeinrichtung in die Kartusche hinein nicht von der Kartusche löst.

Die Befestigung kann über einen Haken oder Stift an der Kartusche erfolgen, insbesondere über einen Stift mit Pilzkopf an der Kartusche erfolgen, der in ein Loch, das in der Trennwand vorgesehen ist, eingesteckt werden kann oder eingesteckt ist. Das rückseitige Ende der Trennwand kann aber auch angeklebt sein oder theoretisch sogar einteilig mit der Kartusche ausgeführt und verbunden sein.

Hiermit wird erreicht, dass sich die Trennwand beim Vortreiben der Umlenkeinrichtung und der Austragskolben nicht vor den Austragskolben, das heißt, zwischen den Austragskolben und dem Kartuschenkopf, faltet und dadurch die Bewegung der Austragskolben in die Hohlräume hinein durch den Faltenwurf der Trennwand behindert wird.

Dabei kann vorgesehen sein, dass am rückseitigen Ende der Trennwand ein Befestigungselement angeordnet ist, wobei das Befestigungselement an einem Gegenbefestigungselement im Bereich der Rückseite der Kartusche zu befestigen ist, wobei vorzugsweise ein Loch in der Trennwand als Befestigungselement und ein Haken oder ein Stift als Gegenbefestigungselement vorgesehen sind.

Hiermit werden besonders einfache und kostengünstig zu realisierende Befestigungsmittel und Gegenbefestigungsmittel bereitgestellt.

Gemäß einer bevorzugten Weiterbildung der vorliegenden Erfindung kann vorgesehen sein, dass in der Umlenkeinrichtung eine Durchführung angeordnet ist oder die Umlenkeinrichtung mit der Innenwand der Kartusche eine Durchführung bildet, wobei die Ablenkfläche in der Durchführung angeordnet ist und die Trennwand durch die Durchführung zu fädeln ist oder durch die Durchführung gefädelt ist, wobei sich die Trennwand durch die Durchführung bewegt, wenn sich die Umlenkeinrichtung in die Kartusche hinein bewegt.

Dadurch werden eine stabile und reproduzierbare Umlenkung und Umformung der Trennwand durch eine Führung der Trennwand in der Durchführung der Umlenkeinrichtung ermöglicht. Bevorzugt wird die Trennwand in der Durchführung im Bereich der Ablenkfläche senkrecht zur Fläche der Trennwand abgelenkt und/oder um eine Achse parallel zur Achse der Kartusche gekrümmt.

Es kann bevorzugt auch vorgesehen sein, dass ein hinterer Teil der Trennwand, der hinter der Vorderseite der Austragskolben beginnt, als Streifen ausgeformt ist, der nicht mit der Innenwand der Kartusche verbunden ist. Dieser Streifen kann besonders bevorzugt durch die Umlenkeinrichtung gefädelt werden oder durch die Umlenkeinrichtung gefädelt sein. Die Vorderseite der Austragskolben ist die dem Kartuschenkopf beziehungsweise dem Austragsrohr zugewandte Seite der Austragskolben, die die beiden Hohlräume mit den Zementkomponenten darin begrenzt.

Es ist vorgesehen, dass die Trennwand mit der Innenwand der Kartusche über eine Sollbruchstelle verbunden ist oder die Trennwand mit der Innenwand der Kartusche lösbar verbunden ist. Hierdurch ist die Trennwand leicht von der Innenwand der Kartusche zu entfernen. Die Trennwand ist nicht einteilig mit der Kartusche ausgeführt, wenn sie lösbar mit der Innenwand der Kartusche verbunden ist. Die Trennwand wird beim Verformen durch die Umlenkeinrichtung nicht von der Umlenkeinrichtung geschnitten. Es findet beim Verformen also keine Trennung eines zuvor zusammenhängenden Materials statt, wenn die Trennwand von der Innenwand der Kartusche lösbar ist.

Die Trennwand löst sich von der Innenwand der Kartusche wenn die Trennwand durch die Bewegung der Umlenkeinrichtung in die Kartusche hinein verformt wird.

Hiermit wird erreicht, dass sich die Trennwand ohne großen Kraftaufwand von der Innenseite der Kartusche lösen lässt, aber gleichzeitig zuvor eine ausreichende Abdichtung der beiden Hohlräume vorhanden ist, so dass die Zementkomponenten in den Hohlräumen gelagert werden können.

Dabei kann vorgesehen sein, dass die Trennwand an beiden seitlichen Rändern mit jeweils einem Führungselement in oder an der Innenwand der Kartusche lösbar verbunden ist, vorzugsweise in jeweils eine Nut in der Innenwand der Kartusche eingesteckt ist, wobei das Führungselement und die seitlichen Ränder der Trennwand fluiddicht miteinander abschließen.

Dadurch kann die Trennwand ohne Aufbrechen einer Sollbruchstelle von der Innenwand der Kartusche gelöst werden. Gleichzeitig kann über das Führungselement beziehungsweise die Nut und die eingesteckte Trennwand zuvor die notwendige Dichtigkeit zwischen den beiden Hohlräumen erreicht werden, damit die beiden Zementkomponenten nicht frühzeitig miteinander reagieren.

Die Trennwand greift vorzugsweise formschlüssig in die zwei Führungselemente ein.

Es kann vorgesehen sein, dass in der Nut oder an dem Rand der Trennwand, der in die Nut eingesteckt ist eine Dichtung vorgesehen ist, mit der die Hohlräume gegeneinander abgedichtet sind.

Ferner kann vorgesehen sein, dass die Austragskolben an der dem Kartuschenkopf gegenüberliegenden Rückseite über die Umlenkeinrichtung miteinander verbunden oder verbindbar sind, vorzugsweise die Austragskolben über die Umlenkeinrichtung derart voneinander beabstandet sind, dass der zwischen den Austragskolben liegende Spalt kleiner oder gleich groß ist, wie die Stärke der Trennwand.

Hiermit wird sichergestellt, dass die Austragskolben stabil in den Hohlräumen laufen, nicht verkannten und gegenüber der Trennwand dicht abschließen, so dass die Zementkomponenten nicht nach hinten aus der Kartusche fließen und so die Umgebung kontaminieren. Durch die Begrenzung des Spalts bezüglich der Stärke der Trennwand wird erreicht, dass die Austragskolben stabil in den Hohlräumen laufen und dass die Trennwand gut durch die Umlenkeinrichtung geführt und umgeformt werden kann.

Es kann erfindungsgemäß vorgesehen sein, dass die Trennwand mit der Mantelfläche des zylindrischen Innenraums der Kartusche entlang zwei Verbindunglinien anliegt, die die Mantelfläche begrenzen, wobei bevorzugt die Verbindungslinien gegenüberliegend zueinander angeordnet sind und besonders bevorzugt die Achse der Kartusche in der Trennwand liegt. Sofern die beiden Nuten vorhanden sind, verlaufen diese entlang dieser Verbindungslinien. Die Verbindungslinien verlaufen bevorzugt axial bezüglich der Kartusche.

Hierdurch können ein gleichmäßiges Vortreiben der Austragskolben in den Hohlräumen und ein gleichmäßiges Überfahren beziehungsweise Umformen der Trennwand durch die Umlenkeinrichtung mit gleichbleibender Kraft erfolgen.

Gemäß einer bevorzugten Ausführungsform der vorliegenden Erfindung kann vorgesehen sein, dass die Hohlräume einen halbkreisförmigen oder kreissegmentförmigen Querschnitt aufweisen und die Austragskolben einen dazu passenden Querschnitt aufweisen, so dass die Austragskolben die Hohlräume in jeder axialen Position in den Hohlräumen verschließen.

Hiermit wird ein besonders einfaches und kostengünstig zu realisierendes Lagerungs- und Mischsystem ermöglicht.

Des Weiteren kann vorgesehen sein, dass die Trennwand eine Stärke von maximal 1,5 mm hat, bevorzugt von maximal 1,0 mm, und/oder die Trennwand eine solche Stärke hat, dass die Trennwand mit der Umlenkeinrichtung, auf die eine Vortriebskraft von 1 kN einwirkt, umzuformen und in Richtung der Innenwand der Kartusche zu drücken ist.

Hiermit wird sichergestellt, dass die Trennwand, wenn sie aus üblichen Kunststoffen hergestellt ist, problemlos mit manuell angetriebenen Austragsvorrichtungen, die auf die Umlenkeinrichtung einwirken, umgeformt und in Richtung der Innenwand der Kartusche gedrückt werden, während die Zementkomponenten von den Austragskolben aus den Hohlräumen ausgepresst werden.

Bevorzugte Lagerungs- und Mischsysteme können sich dadurch auszeichnen, dass das Lagerungs- und Mischsystem ein Austragsrohr aufweist, an dem ein Befestigungsmittel zur Befestigung des Austragsrohrs an der Kartusche vorgesehen ist, wobei vorzugsweise das Austragsrohr anstelle des Kartuschenkopfs an der Kartusche zu befestigen ist.

Das Befestigungsmittel ist bevorzugt ein Innengewinde, das auf ein Außengewinde an der Kartusche geschraubt werden kann. Besonders bevorzugt wird das Außengewinde an der Kartusche auch zur lösbaren Befestigung des Kartuschenkopfs an der Kartusche verwendet.

Im Austragsrohr ist erfindungsgemäß bevorzugt ein statischer Mischer angeordnet. Mit der Erfindung wird auch vorgeschlagen, dass im Austragsrohr ein statischer Mischer angeordnet ist und dass an der Basis des Austragsrohrs als Verbindungsmittel ein Innengewinde, ein Außengewinde, Elemente eines Bajonettverschlusses und/oder Rastelemente eines Rastverschlusses angebracht sind.

Hiermit kann das Austragsrohr dazu verwendet werden, die Zementkomponenten zu mischen und punktgenau zu applizieren. Insbesondere bei schwer zugänglichen Orten zur Applikation eines PMMA-Knochenzements ist ein längeres Austragsrohr vorteilhaft.

Als statische Mischer kommen alle allgemein bekannten statischen Mischer in Betracht. An der Basis des Austragsrohrs sind als Verbindungsmittel ein Innengewinde und/oder ein Außengewinde und/oder Elemente eines Bajonettverschlusses und/oder Rastelemente eines Rastverschlusses angebracht. Mit diesem Verbindungsmittel kann das Austragsrohr mit der Kartusche mechanisch stabil verbunden werden. Diese Verbindung muss stabil sein, damit der bei dem Herauspressen der pastenförmigen Zementkomponenten auftretende hohe Druck nicht zu einem Ablösen des Austragsrohrs von der Kartusche führt. Besonders vorteilhafte Verbindungsmittel sind dabei Gewinde und ganz besonders vorteilhaft sind Doppelgewinde.

Bei Lagerungs- und Mischsystemen mit Austragsrohr kann zudem vorgesehen sein, dass das Verhältnis des Durchmessers des Innenraums der Kartusche zum Innendurchmesser des Austragsrohrs kleiner als 5 zu 2 ist, wobei bevorzugt das Verhältnis des Durchmessers des Innenraums der Kartusche zum Innendurchmesser des Austragsrohrs kleiner oder gleich 2 zu 1 ist und ganz besonders bevorzugt das Verhältnis des Durchmessers des Innenraums der Kartusche zum Innendurchmesser des Austragsrohrs 8 zu 5 ist.

Hierdurch wird erreicht, dass während des Vortreibens der Austragskolben eine ausreichende Strömungsgeschwindigkeit des PMMA-Knochenzements an der Austragsöffnung des Austragsrohrs erreicht wird.

Es kann insbesondere auch vorgesehen sein, dass der Durchmesser des Innenraums der Kartusche kleiner oder gleich 25 mm ist, wobei bevorzugt der Durchmesser des Innenraums der Kartusche kleiner oder gleich 20 mm ist.

Es kann bei Lagerungs- und Mischsystemen mit Austragsrohr auch vorgesehen sein, dass der Durchmesser des Innenraums der Kartusche kleiner oder gleich 25 mm ist und der Innendurchmesser des Austragsrohrs kleiner oder gleich 15 mm ist, wobei bevorzugt der Durchmesser des Innenraums der Kartusche kleiner oder gleich 20 mm ist und der Innendurchmesser des Austragsrohrs kleiner oder gleich 12 mm ist.

Durch den erfindungsgemäßen Aufbau der Kartusche beziehungsweise der Kartusche und des Austragsrohrs gelingt es, beide pastöse Zementkomponenten des PMMA-Knochenzements in einer einzigen Kartusche unterzubringen, die noch durch manuelle Krafteinwirkung auspressbar ist und die aber auch gleichzeitig noch mit herkömmlichen Techniken befüllt werden kann. Bei größeren Durchmessern reicht eine manuelle Krafteinwirkung nicht mehr ohne weiteres aus, um die zähflüssigen pastösen Zementkomponenten des Knochenzements aus der Kartusche zu pressen. Bei den angegebenen Durchmessern wirken sich die Vorteile der vorliegenden Erfindung also besonders stark aus.

Gemäß einer bevorzugten Weiterbildung der vorliegenden Erfindung kann auch vorgesehen sein, dass in dem Kartuschenkopf zwei Durchführungen vorgesehen sind, die die beiden Hohlräume mit der Umgebung des Lagerungs- und Mischsystems verbinden, wobei in den Durchführungen jeweils ein lösbarer Stopfen angeordnet ist.

Hiermit wird ermöglicht, dass die Zementkomponenten durch die Durchführungen in die Hohlräume eingefüllt werden können. Bei einer Variante der vorliegenden Erfindung können die Zementkomponenten nach Entfernen der Stopfen auch wieder durch die Durchführungen ausgepresst werden. Der Kartuschenkopf ohne die Stopfen verbleibt bei dieser Ausführung auch nach dem Anbringen des Austragsrohrs noch an der Vorderseite des Innenraums der Kartusche angeordnet.

Bei bevorzugten Lagerungs- und Mischsystemen kann auch vorgesehen sein, dass die Kartusche auf der dem Kartuschenkopf gegenüberliegenden Seite ein Befestigungselement für eine Austragsvorrichtung aufweist und auf der Seite des Kartuschenkopfs mindestens ein Befestigungsmittel aufweist, insbesondere ein Außengewinde, ein Innengewinde, mindestens ein Element eines Bajonettverschlusses und/oder ein Rastelement eines Rastverschlusses als Befestigungsmittel besitzt.

Hiermit wird der Anschluss der Kartusche an die Auspressvorrichtung an der Rückseite beziehungsweise an ein Austragsrohr und/oder einen Verschluss des Kartuschenkopfs an der Vorderseite erleichtert.

Die Austragskolben können zur Vereinfachung der Befüllung der Hohlräume mit den Zementkomponenten verwendet werden, wenn sie zunächst an dem Kartuschenkopf anliegen. Die Zementkomponenten werden in die Hohlräume eingepresst und dabei die Austragskolben in Richtung der Rückseite der Kartusche gedrückt, ohne dass im Inneren der Hohlräume unerwünschte Lufteinschlüsse verbleiben, die beim Austreiben der Zementkomponenten mit den Austragskolben aus den Hohlräumen stören würden. Es können Rastmittel vorgesehen sein, die die Austragskolben mit der Umlenkeinrichtung verbinden.

Des Weiteren kann vorgesehen sein, dass die Kartusche, der Kartuschenkopf, die Trennwand, die Umlenkeinrichtung und die Austragskolben aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

Der Aufbau mit Kunststoffen ist kostengünstig und einfach umsetzbar. Die bevorzugten Kunststoffe sind aufgrund ihrer Resistenz gegenüber den in den Zementkomponenten enthaltenen Chemikalien besonders gut geeignet.

Ferner kann vorgesehen sein, dass der Kartuschenkopf durch eine gummielastischen Platte realisiert ist, die mit einer Überwurfkappe an der Kartusche befestigt ist, wobei die Überwurfkappe eine Bewegung der gummielastischen Platte von der Kartusche weg mit Hilfe eines überragenden Rands blockiert, und wobei die gummielastische Platte an der den Austragskolben zugewandten Seite eine Aussparung zur Aufnahme der Breitseite der Trennwand besitzt, und wobei vorzugsweise durch diese Aufnahme in der gummielastischen Platte zwei Bereiche definiert sind, wobei in jedem Bereich eine Durchführung angeordnet ist, die durch einen Stopfen verschlossen ist.

Hiermit wird eine Verbesserung der Dichtwirkung des Lagerungs- und Mischsystems erreicht. Die Teilung der gummielastischen Platte in zwei Bereiche ist nicht so verstehen, dass die gummielastische Platte zwei separate Teile aufweisen muss. Die beiden Bereiche können also zusammenhängend sein und durch eine einteilige gummielastische Platte realisiert sein.

Es kann dabei auch vorgesehen sein, dass der Kartuschenkopf zusätzlich mit einer Kunststoffplatte aufgebaut ist, wobei die Kunststoffplatte auf oder unter der gummielastischen Platte im Kartuschenkopf angeordnet ist. Die Kunststoffplatte dient der zusätzlichen Abdichtung und fördert die chemische Beständigkeit der damit begrenzten Hohlräume gegen die Zementkomponenten. Die Anordnung der zusätzlichen Kunststoffplatte führt zu einer verbesserten Diffusionsbarriere gegenüber dem in den Zementkomponenten enthaltenen Methylmethacrylat.

Ferner kann vorgesehen sein, dass eine Überwurfkappe als Verbindungselement zur Verbindung des Kartuschenkopfs mit der Kartusche vorgesehen ist, wobei die Überwurfkappe ein Innengewinde oder ein Außengewinde oder ein Bajonettverschluss oder Rastelemente aufweist.

Die Überwurfkappe ist bevorzugt eine Überwurfmutter und kann auf die Kartusche aufgeschraubt werden. Die Überwurfkappe kann als Teil des Kartuschenkopfs aufgefasst werden. Hierdurch kann der Kartuschenkopf stabil mit der Kartusche verbunden werden. Mit dem Verbindungselement kann die Überwurfkappe sicher mit der Kartusche verbunden werden. Eine Ablösung der Kartuschenkopfs von der Kartusche währender Lagerung und des Transports kann dadurch sicher verhindert werden.

Gemäß einer besonders bevorzugten Ausführung der vorliegenden Erfindung kann vorgesehen sein, dass die Umlenkeinrichtung auf der dem Kartuschenkopf abgewandten Rückseite eine seitliche Öffnung aufweist, so dass die Trennwand durch diese Öffnung seitlich aus der Umlenkeinrichtung austritt.

Hierdurch wird erreicht, dass die Trennwand durch die Öffnung geführt beziehungsweise geleitet wird und so sicher in Richtung der Innenwand der Kartusche gelenkt beziehungsweise gedrückt wird, damit die Trennwand die Bewegung des Stößels der Austragsvorrichtung, mit der die Austragskolben und die Umlenkeinrichtung angetrieben werden, nicht stört beziehungsweise in seiner Bewegung hemmt. Zudem kann die durch die Öffnung in der Umlenkeinrichtung gebildete Durchführung auch dazu verwendet werden, den zumindest einen abgeschnittenen Streifen der Trennwand innerhalb der Umlenkeinrichtung umzuformen.

Zudem kann vorgesehen sein, dass die geneigte Ablenkfläche bereichsweise von einer Wandung der Umlenkeinrichtung umgeben ist, so dass die durch die Umlenkeinrichtung laufende Trennwand von der die geneigte Ablenkfläche umgebenden Wandung der Umlenkeinrichtung um die Längsachse der Trennwand gekrümmt wird.

Die Wandung kann so zum Umformen der Trennwand verwendet werden, so dass diese aufgrund ihrer Form leichter an die Innenwand der Kartusche angelegt werden kann, beziehungsweise aufgrund der Form die Gefahr für eine ungewollte Beeinträchtigung des Antriebs über den Stößel der Austragsvorrichtung reduziert ist.

Ferner kann vorgesehen sein, dass die Umlenkeinrichtung als offener Hohlkörper ausgebildet ist, wobei eine Öffnung an der von den Austragskolben abgewandten Rückseite des Hohlkörpers bogenförmig der Innenkontur der Kartusche folgt, wobei die Bogenlänge größer oder gleich der Breite der Trennwand ist.

Hiermit wird erreicht, dass die Umlenkeinrichtung beziehungsweise der offene Hohlkörper die Trennwand derart umformt, dass eine ungewollte Behinderung des Vortriebs des Stößels der Austragsvorrichtung, die zum Vortreiben der Austragskolben und der Umlenkeinrichtung beziehungsweise dem offenen Hohlkörper verwendet wird, verhindert wird.

Dabei kann vorgesehen sein, dass eine Verlängerung der Trennwand im Bereich der dem Kartuschenkopf gegenüberliegenden Rückseite der Trennwand durch die rückseitige Öffnung austritt und die Trennwand an diesem Ende an mindestens einem Punkt der Kartusche fixiert ist.

Damit wird verhindert, dass sich die Trennwand zwischen dem Kartuschenkopf und den Austragskolben beziehungsweise zwischen dem Austragsrohr und den Austragskolben ungewollt vorformt.

Bevorzugte Ausführungen der vorliegenden Erfindung können auch vorsehen, dass die Trennwand als Platte ausgebildet ist, die an der Schmalseite mindestens eine umlaufende gummielastische Dichtung besitzt.

Hiermit wird eine bessere Abdichtung der Hohlräume gegeneinander erreicht.

Die der vorliegenden Erfindung zugrundeliegenden Aufgaben werden auch gelöst durch ein Verfahren zur Vermischung von pastenförmigen Zementkomponenten eines pastenförmigen Zementteigs, insbesondere eines Polymethylmethacrylat-Knochenzements, mit einem erfindungsgemäßen Lagerungs- und Mischsystem, gekennzeichnet durch die folgenden nacheinander ablaufenden Schritte,
a) Entfernen des Kartuschenkopfs von der Kartusche oder Entfernen von zumindest zwei Stopfen aus zumindest zwei Durchführungen im Kartuschenkopf, so dass die Kartusche geöffnet wird,
b) Aufsetzen und Verbinden eines Austragsrohrs mit der geöffneten Kartusche, wobei das Austragsrohr einen Mischer enthält,
c) Einsetzen der Kartusche in eine manuell zu bedienende Austragsvorrichtung, die Austragsvorrichtung aufweisend einen axial vortreibbaren Stößel zum Vortreiben der Austragskolben in den Hohlräumen der Kartusche,
d) Auspressen der pastenförmigen Zementkomponenten mit Hilfe der Austragsvorrichtung durch axiales Vortreiben der Austragskolben mit dem Stößel, wodurch die Zementkomponenten in das Austragsrohr gedrückt werden, wobei die beiden Zementkomponenten durch den Mischer im Austragsrohr zu dem pastenförmigen Zementteig gemischt werden und der gemischte Zementteig aus einer Austragsöffnung des Austragsrohrs ausfließt, wobei synchron zur Bewegung der Austragskolben die Umlenkeinrichtung über die Trennwand in die Kartusche eingedrückt wird und die Trennwand mit einer Ablenkfläche der Umlenkeinrichtung in Richtung der Innenwand der Kartusche zumindest derart weit in Richtung der Innenwand der Kartusche gedrückt wird, dass eine weitere Bewegung des Stößels der Austragsvorrichtung nicht durch die Trennwand verhindert wird oder nicht durch die Trennwand behindert wird, wobei die Trennwand durch die sich in Richtung des Kartuschenkopfs bewegende Umlenkeinrichtung in axialer Richtung gekrümmt wird und durch die Krümmung der Trennwand die Trennwand von der Innenwand der Kartusche gelöst wird.

Die Schritte laufen bevorzugt chronologisch nacheinander ab. Die Austragsvorrichtung wird vorzugsweise manuell angetrieben.

Vorzugsweise wird die Trennwand derart in Richtung der Innenwand gedrückt und derart umgeformt, dass sie an der Innenwand der Kartusche anliegt, nachdem die Umlenkeinrichtung über die Trennwand hinweg gedrückt wurde.

Vor dem Entfernen des Kartuschenkopfs von der Kartusche oder dem Entfernen der zumindest zwei Stopfen aus den zumindest zwei Durchführungen im Kartuschenkopf ist die Kartusche beziehungsweise die Hohlräume der Kartusche, in denen die Zementkomponenten lagern, verschlossen.

Es ist vorgesehen, dass die Trennwand durch die sich in Richtung des Kartuschenkopfs bewegende Umlenkeinrichtung in axialer Richtung gekrümmt wird und dass durch die Krümmung der Trennwand die Trennwand von der Innenwand der Kartusche gelöst wird, vorzugsweise aus den Führungselementen herausgelöst wird.

Hiermit wird erreicht, dass die Umlenkeinrichtung die Trennwand ohne großen Kraftaufwand zunächst von der Innenwand der Kartusche lösen kann und anschließend die Umlenkung der gelösten Trennwand über die Ablenkfläche ebenfalls ohne großen Kraftaufwand erfolgen kann. Die Reduzierung des notwendigen Kraftaufwands führt dazu, dass auch einfache manuell angetriebene Austragsvorrichtungen zum Antreiben des erfindungsgemäßen Lagerungs- und Mischsystems verwendet werden können.

Bei erfindungsgemäßen Verfahren kann ferner vorgesehen sein, dass eine Fixierung die Trennwand an der Rückseite der Kartusche derart fixiert, dass eine axiale Bewegung der Trennwand in den Führungselementen vor den Austragskolben verhindert wird.

Hiermit wird verhindert, dass die Trennwand zwischen den Austragskolben und dem Kartuschenkopf Falten schlägt oder sich dort verformt und dadurch die Bewegung der Austragskolben in die Hohlräume hinein behindert oder erschwert. Alternativ könnte auch vorgesehen sein, dass sich die Trennwand an Sollknickstellen verformt und nicht an der Rückseite der Kartusche befestigt ist. Die Trennwand wird dann zwischen den Austragskolben und dem Kartuschenkopf beziehungsweise dem Austragsrohr gefaltet und dabei die Zementkomponenten ausgetrieben. Diese Variante ist aber nachteilig, da eine definierte Faltung vor den Austragskolben aufwendiger zu realisieren ist, als die nur leichte durchlaufende Umformung der Trennwand mit Hilfe der Umlenkeinrichtung.

Es kann erfindungsgemäß vorgesehen sein, dass zur Entfernung des Kartuschenkopfs von der Kartusche in Schritt a) ein Verbindungselement gelöst wird, das den Kartuschenkopf mit der Kartusche verbindet.

Hierdurch wird eine stabilere Verbindung zwischen dem Kartuschenkopf und der Kartusche erreicht. Zudem kann das Gegenstück an der Kartusche, das heißt ein Verbindungselement an der Kartusche auch zur Verbindung des Austragsrohrs verwendet werden.

Ferner kann vorgesehen sein, dass das Austragsrohr mit der Kartusche durch Verbindung des Verbindungselements des Austragsrohrs mit einem Verbindungselement der Kartusche verbunden wird.

Hierdurch kann sichergestellt werden, dass sich das Austragsrohr beim Auspressen des Zementteigs nicht von der Kartusche löst.

Des Weiteren kann erfindungsgemäß vorgesehen sein, dass der Stößel der Austragsvorrichtung auf die von den Austragskolben abgewandte Seite der Umlenkeinrichtung drückt und die Austragskolben über die Umlenkeinrichtung angetrieben werden.

Dadurch wird erreicht, dass die durch den Stößel verfügbare Kraft mit einem möglichst großen Anteil zum Antreiben der Zementkomponenten sowie zum Umformen und seitlichen Wegdrücken der Trennwand genutzt werden kann. Es soll dadurch vermieden werden, dass ein zu großer Teil der Kraft in eine ungewollte Verformung der Kartusche oder eine störende Verkantung der Austragskolben fließt.

Es kann auch vorgesehen sein, dass die von den Austragskolben abgewandte Seite der Umlenkeinrichtung eine Anlagefläche zum Anlegen der Vorderseite des Stößels oder eines daran angebrachten Tellers aufweist, die gleich groß oder größer ist als der Querschnitt des Stößels oder als die Auflagefläche des Tellers, wobei beim Vortreiben des Stößels der Querschnitt des Stößels oder die Auflagefläche des Tellers vollständig von der Anlagefläche abgedeckt wird oder vorzugsweise die Anlagefläche den Querschnitt des Stößels oder die Auflagefläche des Tellers überragt.

Hiermit wird erreicht, dass die Bewegung des Stößels nicht von dem zumindest einen abgeschnittenen Streifen der Trennwand behindert wird.

Bevorzugt kann auch vorgesehen sein, dass die Austragsvorrichtung manuell antreibbar ist oder durch Druckluft oder elektrisch antreibbar ist.

Manuell antreibbare Austragsvorrichtungen sind erfindungsgemäß bevorzugt, da sie weder an eine Druckluftquelle oder eine Energiequelle angeschlossen werden müssen, noch eine solche enthalten müssen.

Der Erfindung liegt die überraschende Erkenntnis zugrunde, dass die Zementkomponenten in einer einzigen gemeinsamen Kartusche mit zylindrischem Innenraum gelagert werden können, wenn die Zementkomponenten in der Kartusche über eine Trennwand, die im Innenraum der Kartusche angeordnet ist, voneinander getrennt werden, wobei beide Austragskolben über eine gemeinsame Umlenkeinrichtung angetrieben werden können, mit der die Trennwand seitlich weggedrückt wird, so dass eine weitere Bewegung eines antreibenden Stößels einer Austragsvorrichtung nicht behindert wird. Dadurch können handgetriebene Austragsvorrichtungen mit nur einem Stößel verwendet werden, da die notwendige Kraft aufgrund des erfindungsgemäßen Aufbaus dazu ausreicht, die Zementkomponenten anzutreiben und zu mischen sowie die Trennwand zu verformen und/oder an die Innenwand der Kartusche wegzudrücken, beziehungsweise die notwendige Kraft vollständig in diese drei Vorgänge gesteckt werden kann. Daher ist der Kraftaufwand zum Vortreiben der Zementkomponenten, zum Mischen der Zementkomponenten und zum Wegbiegen der Trennwand aufgrund des erfindungsgemäßen Aufbaus in der Summe nicht so groß, dass die Austragsvorrichtung insgesamt zu schwergängig würde.

Ferner wurde überraschend gefunden, dass auf diese Weise eine schmale Kartusche mit nur einem einzigen Austragskolben zum Vortreiben der beiden Zementkomponenten verwendet werden kann. Dadurch kann die Kraft, die notwendig ist, um die Zementkomponenten zu mischen und auszutreiben, minimiert werden, so dass eine mit manueller Kraft anzutreibende Austragsvorrichtung zusammen mit dem Lagerungs- und Mischsystem eingesetzt werden kann, um die Zementkomponenten aus der Kartusche auszutreiben und miteinander zu mischen.

Die Erfindung basiert auf der Idee, zur Minimierung des Strömungswiderstands beim Austrag nur eine zylindrische Kartusche, anstelle von mehreren side-by-side-Kartuschen oder KoaxialKartuschen, für die separate Lagerung der beiden pastenförmigen Zementkomponenten einzusetzen. Zur Vermeidung von zwei Schubstangen und zwei Tellern zum Antreiben von zwei Austragskolben wird die zylindrische Kartusche dazu mit einer axialen Trennwand ausgerüstet, die den Innenraum der Kartusche, der durch zwei Austragskolben und einen Kartuschenkopf begrenzt ist, in zwei Hohlräume teilt, in denen die beiden pastenförmigen Zementkomponenten separat gelagert werden können. Durch das Wegbiegen beziehungsweise Wegdrücken der Trennwand mit Hilfe der Umlenkeinrichtung können auch kleinere Mengen des PMMA-Knochenzements verwendet werden und auch schmalere Kartuschen mit Innenräumen mit kleineren Innendurchmessern sind noch auspressbar.

In einer ersten Ausführungsform wird der Kartuschenkopf entfernt und das Austragsrohr, das einen statischen Mischer enthält, direkt mit einem Verbindungselement mit der Kartusche verbunden. Unmittelbar anschließend wird die Kartusche mit dem angeschlossenen Austragsrohr mit einer manuell zu betätigenden Austragsvorrichtung beziehungsweise einem manuell zu betätigenden Applikator verbunden und mit dem Austrag der Zementkomponenten beziehungsweise des Zementteigs begonnen. Diese Schritte erfordern einen Zeitaufwand von ungefähr 3 bis 8 Sekunden. Nach ungefähr 30 Sekunden kontinuierlicher Betätigung der Auspressvorrichtung beziehungsweise des Applikators ist bei einem gesamten Volumen beider pastenförmigen Zementkomponenten von maximal 60 ml, vorzugsweise von circa 40 ml, der Austrag des Zementteigs also des gemischtem pastösen Zweikomponenten-PMMA-Knochenzements beendet.

In einer zweiten Ausführungsform werden zwei Stopfen am Kartuschenkopf entfernt und das Austragsrohr mit statischem Mischer mit einem Verbindungselement mit der Kartusche verbunden.

Die Erfindung basiert weiterhin auf der Idee, dass ein im ersten Hohlraum axial beweglich angeordneter erster Austragskolben und im zweiten Hohlraum ein axial beweglicher zweiter Austragskolben angeordnet ist. Zwischen den Austragskolben befindet sich die Trennwand. An der Rückseite der Austragskolben sind diese über eine Umlenkeinrichtung miteinander verbunden oder miteinander verbindbar. Durch die Umlenkeinrichtung verläuft die Trennwand und ist hinter der Umlenkeinrichtung mit der Kartusche verbunden. Auf der Rückseite der Umlenkeinrichtung ist eine Anlagefläche für den Stößel einer Austragsvorrichtung ausgebildet. Bei der Vorwärtsbewegung der Umlenkeinrichtung durch Einwirkung des Stößels in Richtung des Kartuschenkopfs beziehungsweise in Richtung eines aufgesetzten Austragsrohrs wird die Trennwand hinter den Austragskolben von der Innenwand des Hohlzylinders gelöst, umgeformt und an die Innenwand der Kartusche beziehungsweise des Hohlzylinders gedrückt. Dazu wird die Trennwand durch eine Ablenkfläche, die in einem Winkel von etwa 45° gegen die Achse der Kartusche beziehungsweise die Bewegungsrichtung der Umlenkeinrichtung geneigt ist, nach außen zur Innenwand der Kartusche gedrückt. Gleichzeitig wird die Trennwand an der Innenseite der Umlenkeinrichtung gekrümmt, so dass die Krümmung parallel zur Krümmung der Innenseite der Kartusche ist. Die Trennwand tritt dann gekrümmt durch eine seitliche Öffnung an der Rückseite der Umlenkeinrichtung parallel zur Innenseite der Kartusche aus. Dadurch kann der Stößel der Austragsvorrichtung auf die Auflagefläche der Umlenkeinrichtung aufsetzen und über die gesamte Länge des Hohlkörpers die Austragskolben unter Auspressung der pastenförmigen Zementkomponenten in Richtung des Austragsrohrs pressen.

Weiterhin basiert die Erfindung auf der Beobachtung, dass hochviskoser Zementteig aus zylinderförmigen Kartuschen durch ein Austragsrohr mit statischem Mischer mit handelsüblichen manuell anzutreibenden Austragsvorrichtungen beziehungsweise Applikatoren in akzeptabler Zeit und mit akzeptablem, da manuell aufbringbarem Kraftaufwand ausgetragen werden kann, wenn der Austragskolben an seiner Stirnseite einen Durchmesser von maximal 25 mm hat. Mit dem erfindungsgemäßen Aufbau wird ein Kartuschensystem bereitgestellt, das derartig kleine Durchmesser für die Anwendung hochviskoser Zementkomponenten realisieren kann. Dabei kann die Kartusche beziehungsweise können die Hohlräume dennoch ohne zu großen Aufwand mit den Zementkomponenten befüllt werden.

Ein beispielhaftes erfindungsgemäßes Lagerungs- und Mischsystem für pastenförmigen Zweikomponenten-Polymethylmethacrylat-Knochenzement ist zusammengesetzt aus
a) einer röhrenförmigen Kartusche,
b) zwei Führungselementen, die in oder an der Innenwand parallel zur Längsachse der Kartusche angeordnet sind,
c) einer axial in der röhrenförmigen Kartusche angeordneten Trennwand, die formschlüssig in die zwei Führungselemente greift, die in oder an der Innenwand der Kartusche angeordnet sind, wobei die Trennwand den Hohlraum der Kartusche in einen ersten Hohlraum, indem eine erste pastenförmige Zementkomponente angeordnet ist, und einen zweiten Hohlraum teilt, in dem eine zweite pastenförmige Zementkomponente angeordnet ist,
d) einem Kartuschenkopf, der ein Ende der röhrenförmigen Kartusche verschließt, wobei an der Unterseite des Kartuschenkopfs eine Aufnahme für die Stirnseite der Trennwand angeordnet ist,
e) einem Austragsrohr mit einem Befestigungsmittel, zur Befestigung an der Kartusche,
f) einem halbmondförmigen beziehungsweise kreissegmentförmigen ersten Austragskolben, der in dem ersten Hohlraum axial beweglich angeordnet ist,
g) einem halbmondförmigen beziehungsweise kreissegmentförmigen zweiten Austragskolben, der in dem zweiten Hohlraum axial beweglich angeordnet ist,
h) einer Umlenkeinrichtung, die als offener Hohlkörper ausgebildet, die an ihrer Vorderseite mit der Rückseite des ersten Austragskolbens und der Rückseite des zweiten Austragskolbens verbunden ist, wobei der Abstand zwischen dem ersten Austragskolben und dem zweiten Austragskolben gleich oder größer der Dicke der Trennwand ist, und wobei an der Rückseite der Umlenkeinrichtung eine Auflagefläche für den Stößel einer Auspressvorrichtung angeordnet ist,
i) wobei eine seitliche Öffnung an der Rückseite des Hohlkörpers in Richtung des Kartuschenbodens angeordnet ist, die bogenförmig der Innenkontur der Kartusche folgt, wobei die Bogenlänge größer oder gleich der Höhe der Trennwand ist,
j) wobei das dem Kartuschenboden zugewandte Teil der streifenförmigen Trennwand zwischen den Austragskolben angeordnet ist und als ein senkrecht zur Längsachse gekrümmter Streifen aus der Öffnung des Hohlkörper austritt, wobei die Krümmung der streifenförmigen Trennwand der Krümmung des Hohlraums der Kartusche folgt, und
k) wobei die aus der Öffnung des Hohlkörpers austretende, gekrümmte, streifenförmige Trennwand an mindestens einem Punkt an der Innenseite der Kartusche fixiert ist.

An der Rückseite des Hohlkörpers in Richtung des Kartuschenbodens befindet sich eine Öffnung. Diese Öffnung folgt bogenförmig der Innenkontur der Kartusche, wobei die Bogenlänge größer, gleich der Höhe der Trennwand ist. Der dem Kartuschenboden zugewandte Teil der streifenförmigen Trennwand der zwischen dem ersten Austragskolben und dem zweiten Austragskolben an der Rückseite der Austragskolben austritt wird durch den Hohlkörper so gekrümmt, dass dieser aus den Führungselementen heraustritt und ein senkrecht zur Längsachse gekrümmter Streifen aus der an der Rückseite angeordneten Öffnung des Hohlkörpers austritt. Dabei folgt die Krümmung der streifenförmigen Trennwand der Krümmung des Hohlraums der Kartusche folgt. Die austretende, gekrümmte streifenförmige Trennwand ist an mindestens einem Punkt an der Innenseite der Kartusche fixiert. Dadurch wird bei einer Vorwärtsbewegung der Umlenkeinrichtung in Richtung des Kartuschenkopfs ein Zusammenfalten der Trennwand verhindert. Beim Auspressen der beiden pastenförmigen Zementkomponenten durch die im Querschnitt halbmondförmigen beziehungsweise kreissegmentförmigen Austragskolben wird die Trennwand hinter den Austragskolben durch die als Hohlkörper ausgebildete Umlenkeinrichtung herausgelöst, gekrümmt und an die Innenseite der Kartusche gepresst, so dass der Stößel ungehindert die Umlenkeinrichtung und damit die Austragskolben in Richtung Kartuschenkopf beziehungsweise in Richtung des Austragsrohrs pressen kann.

Erfindungsgemäß kann vorgesehen sein, dass axiale Aussparungen an der Innenseite der Kartusche als Führungselemente angeordnet sind. Alternativ können auch axiale Stege oder Vorsprünge an der Innenwand der Kartusche als Führungselemente für die Trennwand vorgesehen sein.

Auf der oberen Stirnseite des Kartuschenkopfs ist bevorzugt eine linienförmige Aussparung angeordnet, wobei die Enden der Aussparungen an den beiden Aussparungen der Kartusche aneinander liegen. Erfindungsgemäß kann alternativ auch ein Steg auf der oberen Stirnseite des Kartuschenkopfs angeordnet sein, dessen Enden mit den axialen Stegen der Kartusche aneinander liegen.

Die Trennwand ist beispielsweise als Platte ausgebildet, die an der Schmalseite mindestens eine umlaufende gummielastische Dichtung besitzt.

Die Trennwand trennt den durch die Innenwand der Kartusche, den Austragskolben und den Kartuschenkopf gebildeten Hohlraum flüssigkeitsundurchlässig in den ersten Hohlraum und den zweiten Hohlraum.

Die pastenförmigen Zementkomponenten enthalten das sehr flüchtige, radikalisch polymerisierbare Monomer Methylmethacrylat. Für eine Lagerung der Zementkomponenten ist es daher unerlässlich, dass die Kartusche, der Kartuschenkopf, die Trennwand und der Austragskolben aus Kunststoffen gefertigt sind, die eine gute Diffusionsbarriere für Methylmethacrylat darstellen. Erfindungsgemäß kann daher vorgesehen sein, dass die Kartusche, der Kartuschenkopf, die Trennwand und der Austragskolben aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden. Es zusätzlich auch möglich, auf die nicht die Zementkomponenten berührenden Teile diffusionsdichte Metallschichten, Metall- oder Halbmetalloxidschichten oder Kunststoffschichten aufzubringen. Als Metallschichten kommen insbesondere Aluminiumschichten in Betracht. Als Halbmetalloxidschichten sind insbesondere Siliziumdioxid-Schichten geeignet.

Die Kartusche hat beispielsweise an einem Ende ein Befestigungselement für eine Auspressvorrichtung und am gegenüberliegenden Ende an der mindestens ein Außengewinde und/oder ein Innengewinde und/oder mindestens eines Elements eines Bajonettverschlusses und/oder ein mindestens ein Rastelement eines Rastverschlusses als Verbindungselement.

Der Kartuschenkopf kann aus einer gummielastischen Platte und einer aus Kunststoff gefertigten Überwurfkappe gebildet sein, wobei die Überwurfkappe die gummielastische Platte nach oben durch einen überragenden Rand blockiert, und wobei in der gummielastische Platte zwei Öffnung angeordnet sind, die durch einen Stopfen verschlossen ist.

In einer ersten Ausgestaltungsform sind eine Kunststoffplatte mit zwei mit Stopfen verschließbare Öffnungen auf oder unter der gummielastischen Platte im Kartuschenkopf angeordnet.

Die Überwurfkappe besitzt als Verbindungselement ein Innengewinde oder ein Außengewinde oder ein Bajonettverschluss oder Rastelemente für einen rastenden Verschluss.

Erfindungsgemäß ist in einem Austragsrohr ein statischer Mischer angeordnet. An der Basis des Austragsrohrs ist als Verbindungsmittel ein Innengewinde und/oder ein Außengewinde und/oder Elemente eines Bajonettverschlusses und/oder Rastelemente eines Rastverschlusses angebracht.

In einer alternativen Ausgestaltungsform ist die Stirnfläche der Kartusche als Kartuschenkopf ausgebildet, wobei zwei durch Stopfen verschließbare Öffnungen die Stirnfläche der Kartusche durchbrechen.

Erfindungsgemäß ist beispielsweise auch ein Verfahren zur Vermischung der pastenförmigen Zementkomponenten des pastenförmigen Polymethylmethacrylat-Knochenzements mit dem Lagerungs- und Mischsystem. Das Verfahren ist durch folgende nacheinander ablaufende Schritte gekennzeichnet:
a) Entfernung des Kartuschenkopfs von der Kartusche oder Öffnen der Kartusche durch Entfernen wenigstens eines Stopfens im Kartuschenkopf,
b) Verbinden eines Austragsrohrs, das einen statischen Mischer enthält, mit der geöffneten Kartusche,
c) Verbinden der Kartusche mit einer manuell zu betätigenden Auspressvorrichtung,
d) manuelle Betätigung der Auspressvorrichtung, wobei der Stößel auf die Auflagefläche drückt, wobei die pastenförmige erste Zementkomponente aus dem ersten Hohlraum durch den im Querschnitt kreissegmentförmigen oder halbmondförmigen ersten Austragskolben und die pastenförmige zweite Zementkomponente aus dem zweiten Hohlraum durch den im Querschnitt kreissegmentförmigen oder halbmondförmigen zweiten Austragskolben in das Austragsrohr und den statischen Mischer gepresst wird, wobei der gemischte Zementteig an der Austragsöffnung des Austragsrohrs austritt,
e) wobei synchron zur Vorwärtsbewegung der Austragskolben parallel zur Trennwand der Hohlkörper als Umlenkeinrichtung hinter den Austragskolben die Trennwand aus den Führungselementen durch Krümmung der Trennwand heraus löst, wobei die gekrümmte Trennwand parallel zur Innenseite der Kartusche neben der Auflagefläche an der Innenseite der Kartusche anliegt, und
f) wobei die Fixierung am Kartuschenboden die aus den Führungselementen herausgetrennte Trennwand so fixiert, dass eine axiale Bewegung der Trennwand in den Führungselementen vor den Austragskolben verhindert wird.

In einer Variante dieses Verfahrens wird in den Schritten d) und e) anstelle der manuell angetriebenen Auspressvorrichtung eine durch Druckluft oder durch elektrischen Strom angetriebene Auspressvorrichtung verwendet.

Im Folgenden werden weitere Ausführungsbeispiele der Erfindung anhand von dreizehn schematisch dargestellten Figuren erläutert, ohne jedoch dabei die Erfindung zu beschränken. Dabei zeigt:
Figur 1: eine schematische perspektivische Querschnittansicht eines erfindungsgemäßen Lagerungs- und Mischsystems;
Figur 2: eine schematische perspektivische Querschnittansicht durch das erfindungsgemäße Lagerungs- und Mischsystem nach Figur 1 unmittelbar vor der Applikation des PMMA-Knochenzements, bei dem ein Austragsrohr an der Kartusche befestigt ist;
Figur 3: eine schematische perspektivische Explosionsdarstellung von erfindungsgemäßen Lagerungs- und Mischsystemen mit zwei unterschiedlichen alternativen Varianten für Austragsrohre;
Figur 4: eine vergrößerte schematische perspektivische aufgeschnittene Ansicht des Kartuschenbodens des erfindungsgemäßen Lagerungs- und Mischsystems nach den Figuren 1 und 2 mit noch nicht eingeschobener Umlenkeinrichtung;
Figur 5: eine vergrößerte schematische perspektivische aufgeschnittene Ansicht des Kartuschenbodens des erfindungsgemäßen Lagerungs- und Mischsystems nach Figur 4 mit eingerasteten Austragskolben und eingesetzter Umlenkeinrichtung, in die die Trennwand eingefädelt ist;
Figur 6: eine vergrößerte schematische perspektivische aufgeschnittene Ansicht des Kartuschenbodens des erfindungsgemäßen Lagerungs- und Mischsystems nach den Figuren 4 und 5, bei dem die Umlenkeinrichtung mit den Austragskolben verbunden ist
Figur 7: eine vergrößerte schematische perspektivische aufgeschnittene Ansicht des Kartuschenbodens des erfindungsgemäßen Lagerungs- und Mischsystems nach Figur 6, bei dem die Trennwand hinter der Umlenkeinrichtung an der Kartusche fixiert ist;
Figur 8: eine schematische perspektivische aufgeschnittene Ansicht eines Kartuschenbodens eines erfindungsgemäßen Lagerungs- und Mischsystems, das in eine Austragsvorrichtung zur Umsetzung eines erfindungsgemäßen Verfahrens eingesetzt ist;
Figur 9: eine schematische perspektivische aufgeschnittene Ansicht des Kartuschenbodens des erfindungsgemäßen Lagerungs- und Mischsystems nach Figur 8, bei dem der Stößel der Austragsvorrichtung die Umlenkeinrichtung und die Austragskolben nach vorne getrieben hat;
Figur 10: eine schematische Querschnittansicht des Kartuschenbodens des erfindungsgemäßen Lagerungs- und Mischsystems nach Figur 8;
Figur 11: eine schematische perspektivische Querschnittansicht des Kartuschenbodens des erfindungsgemäßen Lagerungs- und Mischsystems nach Figur 8;
Figur 12: eine schematische perspektivische Querschnittansicht auf den vorderen Teil des erfindungsgemäßen Lagerungs- und Mischsystems nach den Figuren 1 und 4 bis 7 und nach den Figuren 8 bis 11; und
Figur 13: eine schematische perspektivische Ansicht auf den vorderen Teil eines alternativen erfindungsgemäßen Lagerungs- und Mischsystems, das mit einem Austragsrohr verbunden ist.

In den Figuren werden der Einfachheit halber für gleiche und gleichartige Bauteile unterschiedlicher Ausführungsformen teilweise die gleichen Bezugszeichen verwendet.

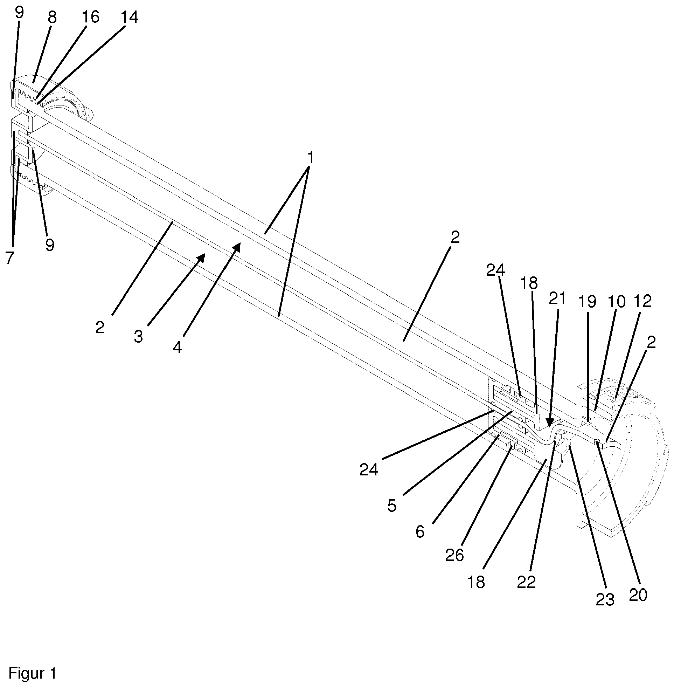

Figur 1 zeigt eine schematische perspektivische Querschnittansicht eines erfindungsgemäßen Lagerungs- und Mischsystems. Das Lagerungs- und Mischsystem weist als zentralen Bestandteil eine zylindrische Kartusche 1 auf, bei der eine Trennwand 2 zwei gegenüberliegende Innenseiten der Innenwand der zylindrischen Kartusche 1 verbindet. Die Kartusche 1 und die Trennwand 2 sind einteilig als gemeinsames Spitzgussteil aufgebaut und die Ränder der Trennwand 2 sind über jeweils eine Sollbruchstelle, wie beispielsweise eine Verjüngung oder eine andere Schwächung des Materials, die sich leicht auftrennen lässt, mit der zylindrischen Innenwand der Kartusche 1 verbunden. Es kann auch vorgesehen sein, dass die Trennwand 2 an der Innenwand der Kartusche 1 anliegt und also nicht einteilig mit der Kartusche 1 verbunden ist. Im letzteren Fall muss allerdings darauf geachtet werden, dass der Rand der Trennwand 2 mit der Innenwand der Kartusche 1 dicht abschließt. Die Trennwand 2 und die Kartusche 1 sind dann zwei separate Teile. Die Trennwand 2 trennt den Innenraum der Kartusche 2 in jedem Fall in zwei separate und fluiddicht voneinander getrennte Hohlräume 3, 4, in denen die beiden pastösen Ausgangskomponenten eines PMMA-Knochenzements lagern.

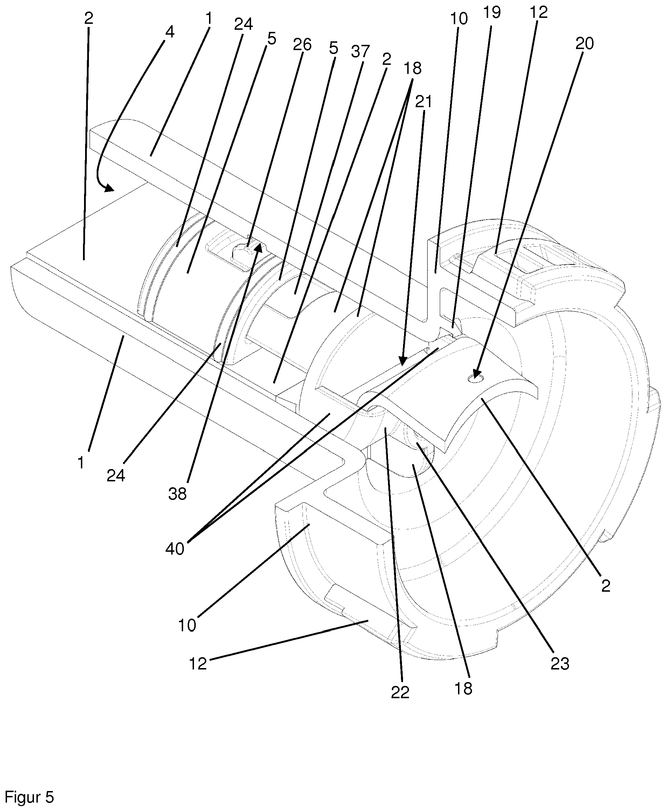

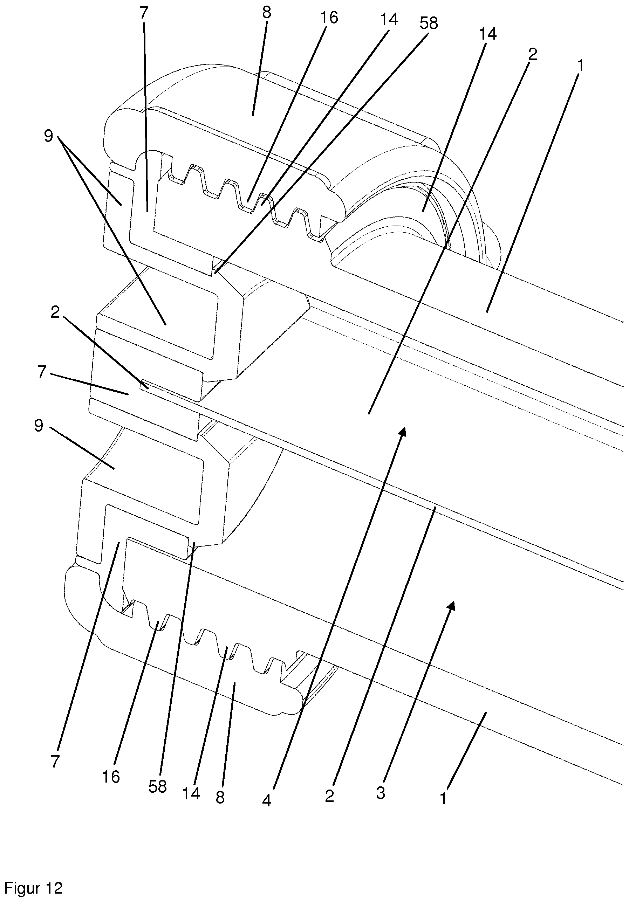

Auf der Rückseite (in Figur 1 rechts) sind die Hohlräume 3, 4 durch zwei Austragskolben 5, 6 begrenzt, wobei die Austragskolben 5, 6 axial in den beiden Hohlräumen 3, 4 verschiebbar gelagert sind. Figur 5 zeigt hierzu eine Detailansicht in Form einer vergrößerten schematischen perspektivischen Teilquerschnittansicht des Kartuschenbodens des erfindungsgemäßen Lagerungs- und Mischsystems nach Figur 1. An der den Austragskolben 5, 6 gegenüberliegenden Vorderseite des Lagerungs- und Mischsystems sind die beiden Hohlräume 3, 4 durch einen Kartuschenkopf 7 in Form einer gummielastischen Platte 7 verschlossen. Der Kartuschenkopf 7 ist mit einer Überwurfmutter 8 aus Kunststoff an der Vorderseite der Kartusche 1 befestigt. In dem Kartuschenkopf 7 sind zwei Durchführungen vorgesehen, die mit jeweils einem Stopfen 9 verschlossen sind. Durch die beiden Durchführungen sind die Hohlräume 3, 4 zugänglich, wenn die Stopfen 9 nicht darin eingesteckt sind. Figur 12 zeigt hierzu eine schematische perspektivische Querschnittansicht auf den vorderen Teil des erfindungsgemäßen Lagerungs- und Mischsystems als Detailansicht, wobei das Lagerungs- und Mischsystem in Figur 12 durch den Kartuschenkopf 7 und die Stopfen 9 darin wie in Figur 1 geschlossen ist.

An der Rückseite der Kartusche 1 beziehungsweise bodenseitig (in Figur 1 rechts) ist an der Kartusche 1 ein Anschluss 10 mit Befestigungselementen 12 angeordnet. Über den Anschluss 10 und die Befestigungselemente 12 kann die Kartusche 1 an eine Austragsvorrichtung beziehungsweise einen Applikator (in Figur 1 nicht gezeigt) angeschlossen werden. An der gegenüberliegenden Vorderseite (in Figur 1 links) der Kartusche 1 ist der Kartuschenkopf 7 mit der Überwurfmutter 8 befestigt, indem auf ein Außengewinde 14 an der Kartusche 1 ein Innengewinde 16 der Überwurfmutter 8 aufgeschraubt ist. Die gummielastische Platte 7 beziehungsweise der Kartuschenkopf 7 dichtet dabei die Hohlräume 3, 4 nach vorne ab.

Die Kartusche 1 hat einen Außendurchmesser von 22 mm, einen Innendurchmesser von 20 mm und eine Länge von etwa 18 cm.

Die beiden Austragskolben 5, 6 sind an deren Rückseite über eine Umlenkeinrichtung 18 miteinander verbunden. Die Umlenkeinrichtung 18 erstreckt sich dazu mit zwei in Richtung des Kartuschenkopfs 7 weisenden zylindersegmentförmigen Enden in passende Hohlräume in den Rückseiten der Austragskolben 5, 6 hinein. Die Trennwand 2 erstreckt sich durch die Umlenkeinrichtung 18 hindurch, beziehungsweise ist durch die Umlenkeinrichtung 18 hindurch gefädelt. Im Bereich des Anschlusses 10 ist an der Kartusche 1 ein pilzförmiger Zapfen 19 mit einer Spitze als Befestigungselement 19 zur Befestigung der Trennwand 2 vorgesehen. In dem hintersten Teil der Trennwand 2, der hinter der Umlenkeinrichtung 18 angeordnet ist (Figur 1 rechts) befindet sich ein Loch 20 in der Trennwand 2. Der Zapfen 19 kann durch das Loch 20 gesteckt werden, um die Trennwand 2 an der Kartusche 1 beziehungsweise am Kartuschenboden beziehungsweise an dem Anschluss 10 der Kartusche 1 zu befestigen. Hiermit wird verhindert, dass sich die Trennwand 2 vor den Austragskolben 5, 6 verformt beziehungsweise Falten wirft, wenn die Austragskolben 5, 6 in den Hohlräumen 3, 4 nach vorne getrieben werden (in Figur 1 nach links).

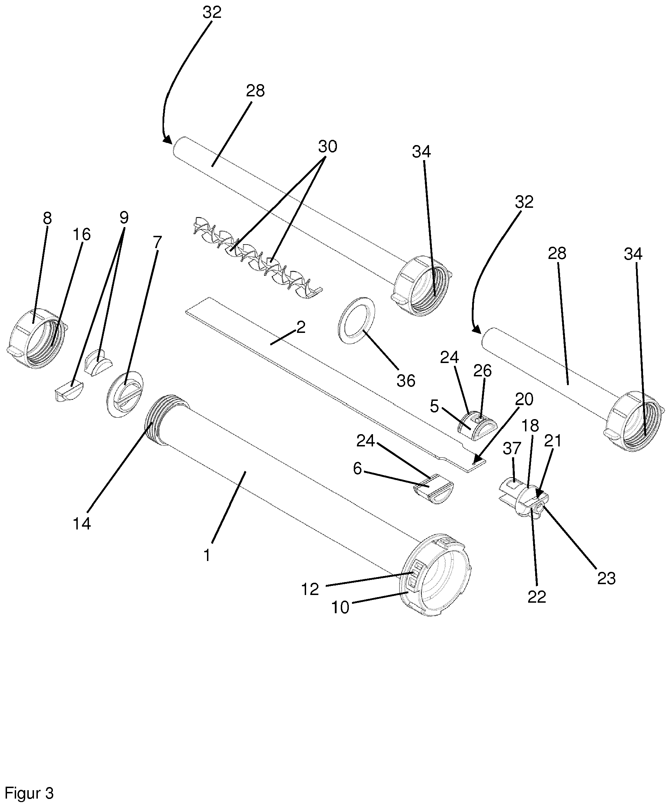

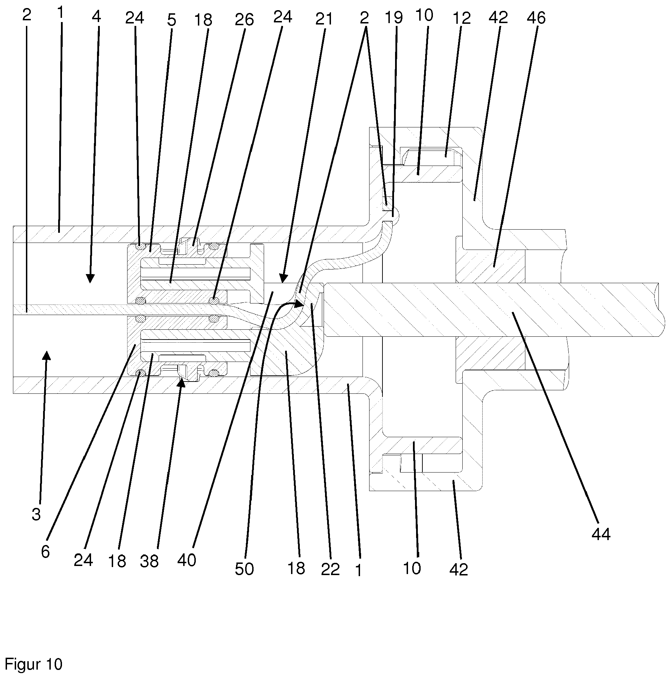

Die Umlenkeinrichtung 18 schließt den Innenraum der Kartusche 1 nach hinten bis auf die Durchführung für die Trennwand 2 ab. Dazu hat die Umlenkeinrichtung 18 bereichsweise eine zylindrische Form (siehe auch Figur 3). Die Trennwand 2 ist durch die Durchführung in der Umlenkeinrichtung 18 geführt und tritt dabei durch eine seitliche Öffnung 21 in der Umlenkeinrichtung 18 aus, die die Trennwand 2 in Richtung der Innenwand der Kartusche 1 führt. An der anderen Seite der Durchführung durch die Umlenkeinrichtung 18 wird die Trennwand 2 durch einen Spalt in die Umlenkeinrichtung 18 eingeführt. Ein Teil der Durchführung durch die Umlenkeinrichtung 18 wird durch eine gegen die Achse der Kartusche 1 und der Trennwand 2 geneigte Ablenkwand 22 begrenzt, die die Trennwand 2 in Richtung der Innenwand der Kartusche 1 ablenkt und dabei umformt. Die Ablenkwand 22 bildet auf der Innenseite in der Durchführung der Umlenkeinrichtung 18 eine geneigte Ablenkfläche (siehe Figur 10, Bezugszeichen 50), die in Richtung des Spalts ausgerichtet ist. Auf der Rückseite der Umlenkeinrichtung 18 ist eine Anlagefläche 23 für einen Stößel einer Austragsvorrichtung (in Figur 1 nicht gezeigt) ausgeformt. Ein Steg der Umlenkeinrichtung 18 sowie das Rohrstück vor der Anlagefläche 23 sorgen für die mechanische Stabilität der Umlenkeinrichtung 18 und können die Kräfte aufnehmen, die beim Umformen der Trennwand 2 entstehen, wenn die Trennwand 2 durch die Umlenkeinrichtung 18 getrieben wird.

Die Austragskolben 5, 6 sind über jeweils zwei umlaufende Dichtungen 24 aus Gummi gegen die Innenwand der Kartusche 1 und gegen die Trennwand 2 abgedichtet. Die Austragskolben 5, 6 sind mit jeweils einem lösbaren Rastelement 26 mit passenden Gegenrastelementen (in Form von zwei Vertiefungen) in der Innenwand der Kartusche 1 verbunden. Die Austragskolben 5, 6 können durch einen Druck von der Rückseite der Kartusche 1 (in Figur 1 von rechts) in Richtung der Vorderseite der Kartusche 1 (in Figur 1 links) also in Richtung des Kartuschenkopfs 7 gedrückt werden. Die Rastelemente 10 können durch einen bodenseitigen Druck auf die Austragskolben 5, 6 ohne weiteres gelöst werden, wenn der Kartuschenkopf 7 oder zumindest die Stopfen 9 entfernt sind. Die Rastelemente 10 dienen vor allem dazu, dass die Austragskolben 5, 6 beim Befüllen der Hohlräume 3, 4 der Kartusche 1 mit den Zementkomponenten nicht bodenseitig aus der Kartusche 1 herausgedrückt werden können, beziehungsweise nicht über die gewünschte, durch die Gegenrastelemente in der Innenwand der Kartusche 1 definierte Position hinaus in Richtung des Kartuschenbodens (in Figur 1 rechts) gedrückt werden können.

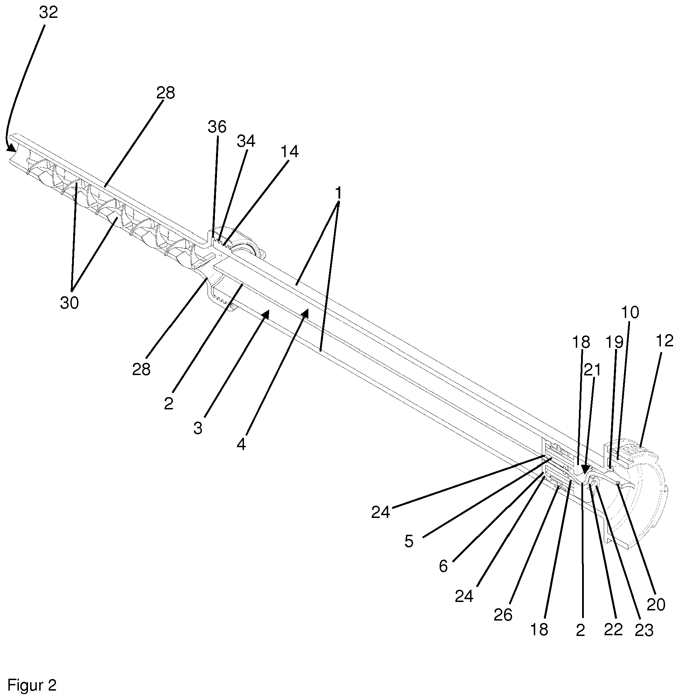

Figur 2 zeigt eine schematische perspektivische Querschnittansicht durch das erfindungsgemäße Lagerungs- und Mischsystem nach Figur 1, unmittelbar vor der Applikation des PMMA-Knochenzements, bei dem ein Austragsrohr 28 auf das Außengewinde 14 der Kartusche 1 aufgeschraubt ist. In dem Austragsrohr 28 ist ein statischer Mischer 30 mit einer Vielzahl von Wendungen und Mischelementen zur Vermischung der Zementkomponenten vorgesehen. Das Austragsrohr 28 kann auch noch länger sein als das in Figur 2 gezeigte Austragsrohr 28, um schwerer zugängliche Bereiche leichter erreichbar zu machen, wie dies beispielsweise bei Operationen an der Hüfte hilfreich sein kann (siehe hierzu beispielsweise Figur 3). In Figur 2 sind alle Teile, bis auf den statischen Mischer 30 geschnitten dargestellt, während der statische Mischer 30 perspektivisch dargestellt aus der Schnittebene herausragt.

Die Zementkomponenten werden gemischt, indem sie durch das Austragsrohr 28 und damit durch den statischen Mischer 30 gedrückt werden. Der so hergestellte und gemischte Zementteig tritt über eine Austragsöffnung 32 an der Spitze des Austragsrohrs 28 aus. Das Austragsrohr 28 weist ein Innengewinde 34 auf, das auf das Außengewinde 14 der Kartusche 1 passt, so dass das Austragsrohr 28 stabil und fest mit der Kartusche 1 verbunden werden kann. Zwischen dem Austragsrohr 28 und der Vorderseite der Kartusche 1 ist ein Dichtungsring 36 angeordnet, damit die Zementkomponenten nicht zwischen dem Austragsrohr 28 und der Kartusche 1 austreten können. Über die Gewinde 14, 34 und den Dichtungsring 36 wird eine druckstabile und druckdichte Verbindung zwischen dem Austragsrohr 28 und der Kartusche 1 erreicht.

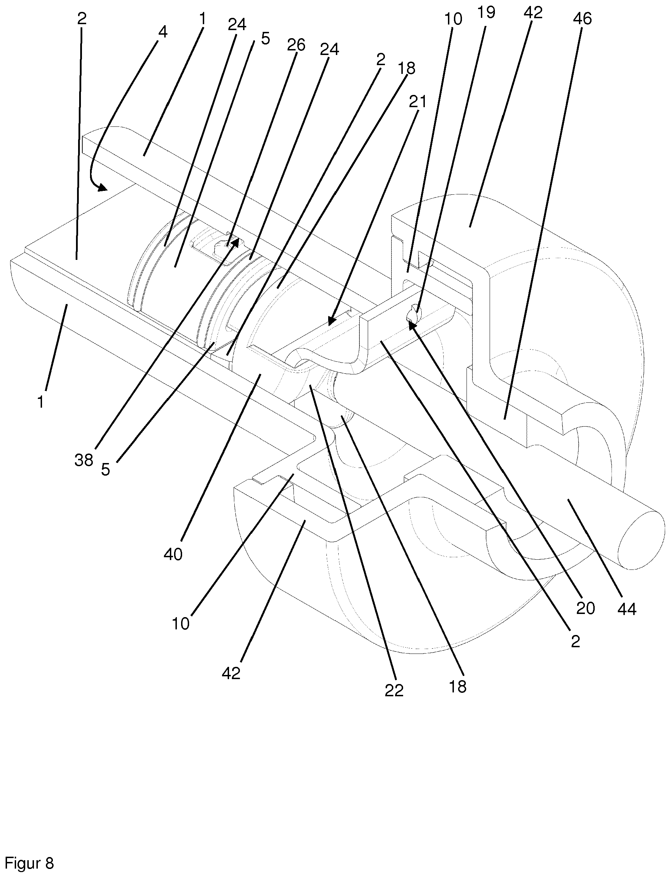

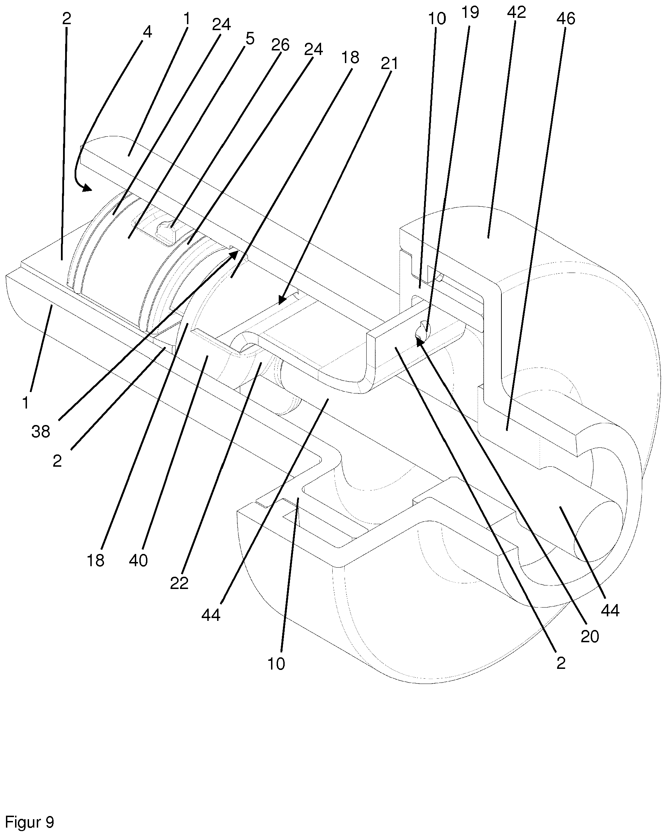

Der Vortrieb der Austragskolben 5, 6 wird über eine Austragsvorrichtung (in Figur 2 nicht gezeigt) erzeugt, die an den Anschluss 12 angeschlossen wird und mit der ein Stößel (siehe Figuren 8 bis 11, Bezugszeichen 44) beziehungsweise eine Schubstange der Austragsvorrichtung manuell in Richtung des Austragsrohrs 28 vorgetrieben werden kann. Der Stößel drückt dann auf die Anlagefläche 23, so dass einerseits die Austragskolben 5, 6 in Richtung des Austragsrohrs 28 vorgetrieben werden und andererseits die Umlenkeinrichtung 18 in die Kartusche 1 getrieben wird, wobei sich die Trennwand 2 durch die Durchführung in der Umlenkeinrichtung 18 schiebt und dabei umgeformt und mit der Ablenkfläche abgelenkt wird (siehe Figur 9). Der Druck des Stößels löst dabei die Rastelemente 10 und treibt die Austragskolben 5, 6 nach vorne. Die Austragskolben 5, 6 schließen dicht mit den Innenwänden der Kartusche 1 und der Trennwand 2 ab. Dadurch kann der Inhalt der Hohlräume 3, 4 der Kartusche 1, nämlich die zwei enthaltenen pastösen Zementkomponenten, nach vorne durch das Austragsrohr 28 ausgetrieben werden.

Figur 3 zeigt eine schematische perspektivische Explosionsdarstellung von erfindungsgemäßen Lagerungs- und Mischsystemen mit zwei alternativen und unterschiedlichen Austragsrohren 28. Der Aufbau der Lagerungs- und Mischsysteme ist analog dem zuvor beschriebenen Lagerungs- und Mischsystem.

In Figur 3 sind zwei alternative Varianten der Austragsrohre 28 mit unterschiedlichen Längen dargestellt. Mit dem längeren Austragsrohr 28 können während einer OP auch schwerer zugängliche Bereiche erreicht werden. Dies kann beispielsweise bei dem Einsetzen einer künstlichen Hüfte vorteilhaft sein.

Der in Figur 3 dargestellte Kartuschenkopf 7 weist zwei kreissegmentförmige beziehungsweise halbmondförmige Durchführungen auf, die durch passende Stopfen 9 verschlossen werden können. In den beiden Durchführungen kann jeweils ein Stopfen 9 eingesteckt und gerastet werden. Die gummielastische Platte 7 und die Stopfen 9 sind zum Aufbau von Kartuschenköpfen 7 erfindungsgemäßer Lagerungs- und Mischsysteme geeignet. Es sind auch andere gummielastische Platten 7 vorstellbar, die sich in der Form ihrer Durchführungen und in der Form der Stopfen 9, die diese Durchführungen verschließen, unterscheiden können. Bei der gezeigten Variante sind die Durchführungen und die Stopfen 9 im Querschnitt halbkreisförmig beziehungsweise halbmondförmig. Bei einer nicht gezeigten Variante können die Durchführungen und die Stopfen 9 im Querschnitt kreisförmig geformt sein. Bei der gezeigten Variante ist der freie Querschnitt zum Einfüllen der beiden Zementkomponenten größer als bei der nicht gezeigten Variante. Dafür ist bei der nicht gezeigten Variante die Geometrie an Einfüllrohre oder Spritzen (ebenfalls nicht gezeigt) angepasst, über die die Zementkomponenten in die Hohlräume 3, 4 eingefüllt werden, so dass die Einfüllrohre beziehungsweise Spritzen dicht mit den Durchführungen abschließen. Die Stopfen 9 können auch entfernt werden, um die Zementkomponenten wieder aus den Hohlräumen 3, 4 der Kartusche 1 auszutragen, wenn nicht der gesamte Kartuschenkopf 7 entfernt werden soll.

An der gummielastischen Platte 7 kann an der in den Innenraum der Kartusche 1 weisenden Seite eine flache Kunststoffscheibe (nicht gezeigt) aufgesetzt sein. Diese Kunststoffscheibe dient einerseits der Stabilisierung der Form der gummielastischen Platte 7 und andererseits der Verbesserung der chemischen Stabilität des Behältnisses beziehungsweise der Hohlräume 3, 4 für die Zementkomponenten.

Der Aufbau der rohrförmigen Kartusche 1 mit der Trennwand 2, dem Anschluss 10, den Befestigungselementen 12 und dem Außengewinde 14, ist bei allen Varianten gleich geformt. Auf das Außengewinde 14 der Kartusche 1 kann sowohl die Überwurfmutter 8 als auch das Austragsrohr 28 aufgeschraubt werden. Es sind auch Varianten denkbar, bei denen die gummielastische Platte 7 fest mit der Kartusche 1 verbunden ist oder auch nicht gummielastisch und/oder einteilig mit der Kartusche 1 ausgeführt ist. Dann wird keine Überwurfmutter 8 benötigt und das Austragsrohr 28 kann nach Entfernen der Stopfen 9 einfach auf die Kartusche 1 aufgeschraubt werden. Die Zementkomponenten können dann einfach durch die Durchführungen in das Austragsrohr 28 gepresst werden und dort mit dem statischen Mischer 30 zu dem gewünschten Zementteig gemischt werden.

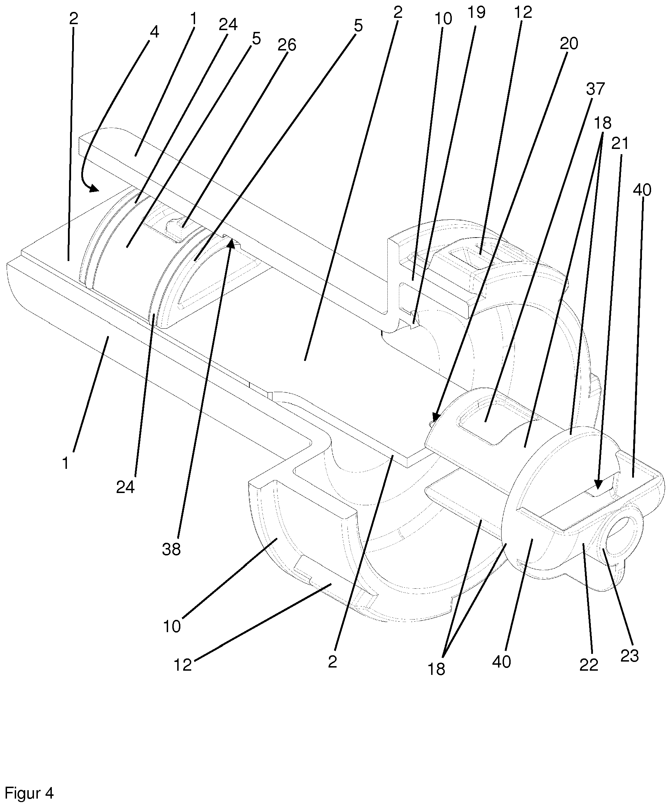

Die Figuren 4 bis 7 zeigen schematische, teilweise aufgeschnittene perspektivische Ansichten und eine Querschnittansicht des Kartuschenbodens des erfindungsgemäßen Lagerungs- und Mischsystems nach den Figuren 1 und 2 in unterschiedlichen Stadien während des Aufbaus der Lagerungs- und Mischeinrichtung. In den beiden zylindersegmentförmigen Schenkeln der Umlenkeinrichtung 18, die in die dafür vorgesehenen Hohlräume in den Austragskolben 5, 6 eingesteckt werden, um diese mit der Umlenkeinrichtung 18 und damit miteinander zu verbinden, sind Vertiefungen 37 als Gegenrastungen 37 vorgesehen, die in Rastungen (nicht gezeigt) im Inneren der Austragskolben 5, 6 greifen. Die Vertiefungen 37 sind insbesondere in den Figuren 4 und 5 gut zu erkennen.

In Figur 4 ist die Umlenkeinrichtung 18 noch nicht bodenseitig in die Kartusche 1 eingesteckt und die Austragskolben 5, 6 noch nicht in ihrer Endposition eingerastet. Die Rastmittel 26 der Austragskolben 5, 6 rasten nämlich in Vertiefungen 38 in der Innenwand der Kartusche 1 ein, wenn sie ausreichend tief in Richtung der Rückseite des Lagerungs- und Mischsystems (in den Figuren 4 bis 7 rechts) in die Hohlräume 3, 4 der Kartusche 1 eingeschoben sind. Die Rastmittel 26 blockieren dabei eine weitere Bewegung der Austragskolben 5, 6 in Richtung der Rückseite des Lagerungs- und Mischsystems, während die Rastung bei einer Bewegung in Richtung des Kartuschenkopfs 7 lösbar ist.

In der in Figur 5 gezeigten Position sind die Austragskolben 5, 6 mit den Rastmitteln 26 in die Vertiefungen 38 eingerastet und die Umlenkeinrichtung 18 ist bodenseitig in die Kartusche 1 eingeschoben, so dass die zylindersegmentförmigen Schenkel der Umlenkeinrichtung 18 bereits ein Stück in die dafür vorgesehenen Öffnungen in den Austragskolben 5, 6 eingeschoben sind. Die Trennwand 2 ist in durch die Durchführung in der Umlenkeinrichtung 18 gefädelt und erstreckt sich durch die seitliche Öffnung 21. Durch das Durchfädeln der Trennwand 2 ist diese umgeformt worden. Zunächst ist die Trennwand 2 senkrecht zu ihrer Mittelachse in Richtung der Innenwand der Kartusche 1 gebogen. Anschließend ist die Trennwand 2 um ihre Achse gekrümmt worden. Die geneigte Ablenkwand 22 ist beidseitig von zwei Wandungen 40 flankiert, die die Ablenkwand 22 seitlich stützen und die seitliche Wandungen 40 der Durchführung bilden. Durch die Wandungen 40 wird die Trennwand 2 um die Achse der Trennwand 2 gekrümmt.

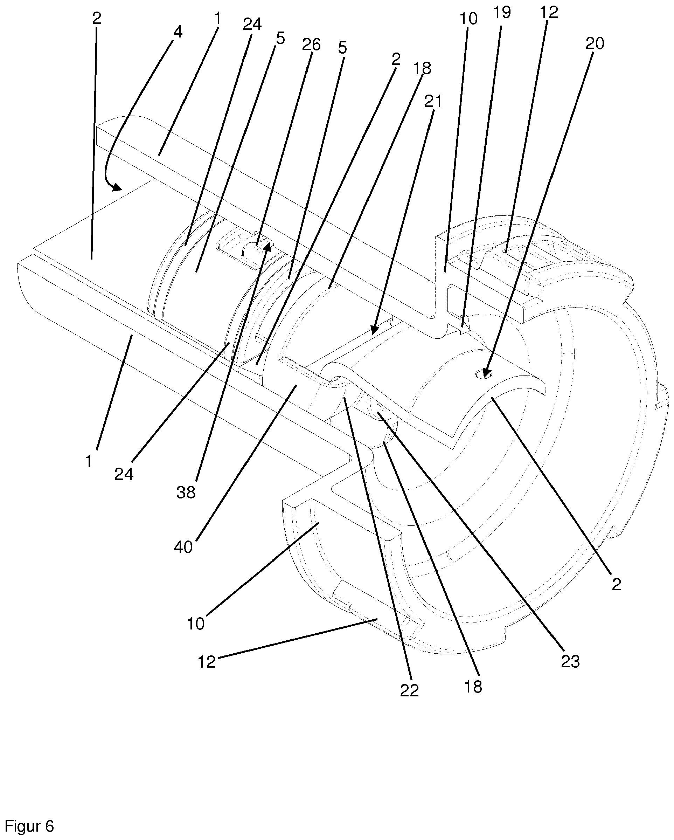

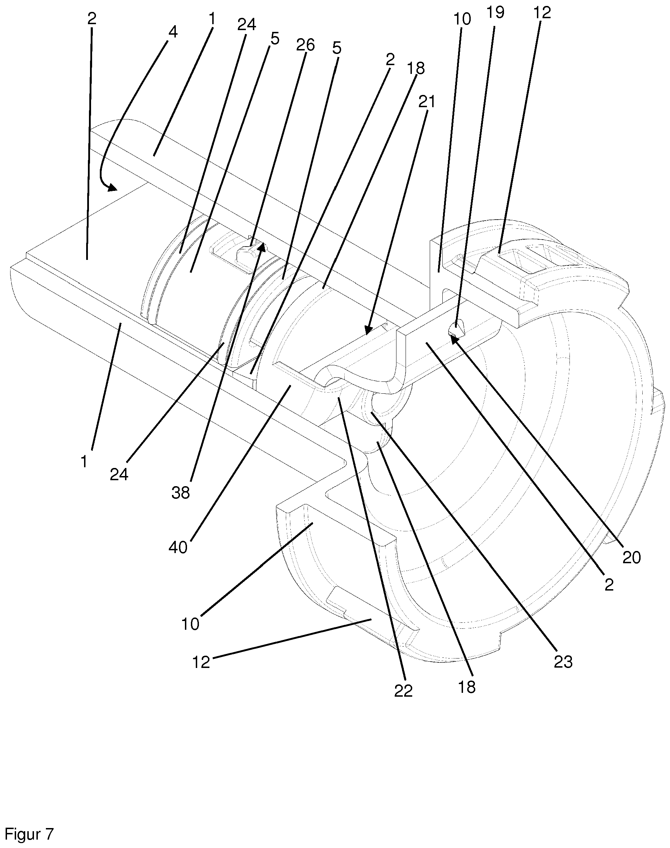

In der in Figur 6 gezeigten Position sind die Austragskolben 5, 6 mit den Rastmitteln 26 in die Vertiefungen 38 eingerastet und die Umlenkeinrichtung 18 ist bodenseitig vollständig in die Austragskolben 5, 6 eingesteckt und verbindet diese. Anschließend kann die Trennwand 2 mit dem Loch 20 auf den Stift 19 gedrückt werden, um die Trennwand 2 an der Kartusche 1 zu fixieren. Dieser Zustand ist in Figur 7 gezeigt. Das Lagerungs- und Mischsystem ist dadurch einsatzbereit. Die Vorderseite des Lagerungs- und Mischsystems ist durch die Kartuschenkopf 7 und die Stopfen 9 verschlossen. In diesem Zustand kann das Lagerungs- und Mischsystem zum Lagern der Zementkomponenten verwendet werden und in diesem Zustand kann es an den Anwender ausgeliefert werden. Dieser Zustand ist daher erfindungsgemäß besonders bevorzugt.

Zum Anwenden des erfindungsgemäßen Lagerungs- und Mischsystems muss dieses nun einsetzbereit gemacht werden. Hierzu ist in den Figuren 8 bis 11 ein erfindungsgemäßes Lagerungs- und Mischsystem dargestellt. Dieses Ausführungsbeispiel entspricht denen nach den vorangehenden Figuren 1 bis 7, wobei als Befestigungsmittel 19 ein Pilzzapfen 19 ohne eine Spitze also mit einer abgerundeten Haube an der Kartusche 1 zur Befestigung der Trennwand 2 angeordnet ist. Da dies nur ein kleiner und unwesentlicher Unterschied ist, kann das im Folgenden erläuterte Verfahren ohne weiteres auf die Ausführungsformen nach den Figuren 1 bis 7, 12 und 13 übertragen werden.

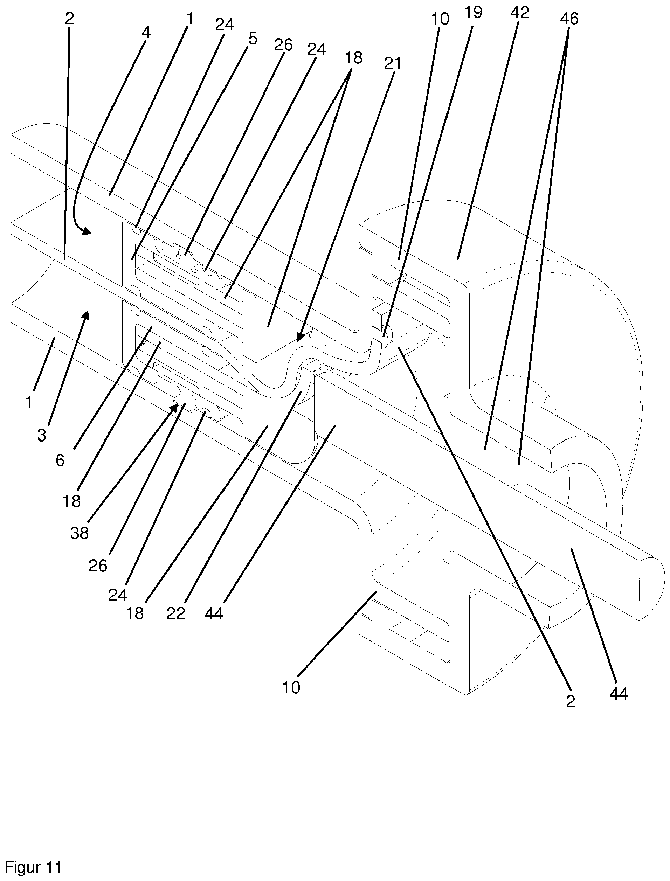

Figur 8 zeigt eine schematische perspektivische aufgeschnittene Ansicht eines Kartuschenbodens eines erfindungsgemäßen Lagerungs- und Mischsystems, das in eine Austragsvorrichtung zur Umsetzung eines erfindungsgemäßen Verfahrens eingesetzt ist, Figur 9 eine schematische perspektivische aufgeschnittene Ansicht des Kartuschenbodens des erfindungsgemäßen Lagerungs- und Mischsystems nach Figur 8, Figur 10 eine schematische Querschnittansicht des Kartuschenbodens des erfindungsgemäßen Lagerungs- und Mischsystems nach Figur 8 und Figur 11 eine schematische perspektivische Querschnittansicht des Kartuschenbodens des erfindungsgemäßen Lagerungs- und Mischsystems nach Figur 8.

Mit den Figuren 8 bis 11 wird ein erfindungsgemäßes Verfahren erläutert, wobei das erfindungsgemäße Verfahren auch ohne weiteres auf die Ausführungsform nach den Figuren 1 bis 7 übertragbar ist. Die Austragsvorrichtung, die zu den Figuren 8 bis 11 erläutert ist, kann ohne weiteres auch bei den Ausführungsformen der Figuren 1 bis 7, 12 und 13 angewendet werden. Der Kartuschenkopf 7 kann getrennt werden oder die Stopfen 9 können aus den Durchführungen im Kartuschenkopf 7 entfernt werden und anstatt der Überwurfmutter 8 wird das Austragsrohr 28 auf die Kartusche 1 aufgeschraubt. In dieser Position kann die Austragsvorrichtung mit dem Anschluss 10 beziehungsweise dem Befestigungsmittel 12 des Lagerungs- und Mischsystems verbunden werden. Dies ist in Figur 8 gezeigt.

Von der Austragsvorrichtung ist in den Figuren 8 bis 11 nur ein Anschluss 42 zum Verbinden mit den Befestigungsmitteln 12 des Lagerungs- und Mischsystems, ein Stößel 44 und eine Lagerung 46 für den Stößel 44 dargestellt. Diese Teile und die restlichen Bauteile der Austragsvorrichtung entsprechen denen üblicher manuell oder elektrisch oder pneumatisch angetriebener Austragsvorrichtungen. Die Austragsvorrichtung hat ein Fach zur Aufnahme des Lagerungs- und Mischsystems, wobei das Lagerungs- und Mischsystem zumindest an der Vorderseite im Bereich des Gewindes 14 und an der Rückseite am Anschluss 10 von der Austragsvorrichtung stabil gehalten wird. Der Anschluss 42 wird mit dem Befestigungsmittel 12 verbunden. Der Stößel 44, der als Schubstange 44 wirkt, kann gegen den Anschluss 42 der Austragsvorrichtung in Richtung durch den Anschluss 42 hindurch beziehungsweise in Richtung in die Kartusche 1 hinein bewegt beziehungsweise angetrieben werden, da er in der Lagerung 46 entlang seiner Längsachse beweglich gelagert ist. Dabei drückt die Spitze des Stößels 44 auf die Anlagefläche 23 der Umlenkeinrichtung 18. Dadurch wird die Umlenkeinrichtung 18 und die beiden Austragskolben 5, 6 in Richtung des Austragsrohrs 28 vorgetrieben.

Die Umlenkeinrichtung 18 wird durch einen Druck auf die Anlagefläche 23 in Richtung des Kartuschenkopfs 7 beziehungsweise des Austragsrohrs 28 vorgetrieben. Die Rastmittel 26 lösen sich aus den Vertiefungen 38 und die Austragskolben 5, 6 werden in den Hohlräumen 3, 4 nach vorne gedrückt. Dabei werden die beiden Zementkomponenten nach vorne in das Austragsrohr 28 gepresst und dort gemischt. Bei einem weiteren Vortreiben der Umlenkeinrichtung 18 werden nicht nur die Austragskolben 5, 6 weiter in den Hohlräumen 3, 4 der Kartusche 1 vorgetrieben, sondern die Trennwand 2 schiebt sich in axialer Richtung durch die Umlenkeinrichtung 18 und wird dabei in der Umlenkeinrichtung 18 abgelenkt und umgeformt sowie hinter den Austragskolben 5, 6 von der Innenwand der Kartusche 1 gelöst. Diese Situation ist in Figur 9 gezeigt, in der dargestellt ist, dass der Stößel 44 der Austragsvorrichtung die Umlenkeinrichtung 18 und die Austragskolben 5, 6 nach vorne getrieben hat. Die Trennwand 2 ist über das Loch 20 mit dem pilzförmigen Stift 19 an der Kartusche 1 befestigt. Dadurch wird verhindert, dass die Trennwand 2 sich einfach im Bereich der Hohlräume 3, 4 verformt und mit den Austragskolben 5, 6 bewegt.

Die Trennwand 2 wird durch den Spalt der Durchführung in der Umlenkeinrichtung 18 zu einer gegen die Achse der Kartusche 1 geneigte Ablenkfläche 50 beziehungsweise zur Ablenkwand 22 geleitet. Durch weiteres Vortreiben der Umlenkeinrichtung 18 drückt die Ablenkfläche 50 beziehungsweise Ablenkwand 22 die Trennwand 2 in Richtung der Innenwand der Kartusche 1. Währenddessen werden die Zementkomponenten weiter aus den Hohlräumen 3, 4 in das Austragsrohr 28 gedrückt und dort gemischt. Schließlich tritt der fertig gemischte Zementteig durch die Austragsöffnung 32 aus dem Austragsrohr 28 aus und kann am gewünschten Ort appliziert werden. In den beiden zylindersegmentförmigen Schenkeln der Umlenkeinrichtung 18, die in die dafür vorgesehenen Hohlräume in den Austragskolben 5, 6 eingesteckt werden, um diese mit der Umlenkeinrichtung 18 und damit miteinander zu verbinden, sind Vertiefungen 37 als Gegenrastungen 37 vorgesehen, die in Rastungen (nicht gezeigt) im Inneren der Austragskolben 5, 6 greifen.

Durch den erfindungsgemäßen Aufbau gelingt es, dass trotz der hohen Zähigkeit der pastösen Zementkomponenten, trotz des Strömungswiderstands, der durch den statischen Mischer 30 verursacht wird und trotz des zum Umformen der Trennwand 2 notwendigen Kraftaufwands beziehungsweise Energieaufwands der Widerstand gegen die Bewegung des Stößels 44 nicht so groß wird, dass die Kartusche 1 nicht mit herkömmlichen, manuell angetriebenen Austragsvorrichtungen auszupressen ist.

Wie in Figur 12 zu erkennen ist, weisen die Stopfen 9 eine Rastung 58 in Form von Vorsprüngen auf, die über die Kante der Durchführungen in Richtung des Innenraums der Kartusche 1 in dem Kartuschenkopf 7 greifen und dadurch mit dem Kartuschenkopf 7 rasten. Durch die Rastung 58 wird erreicht, dass die Stopfen 9 sich nicht ungewollt aus dem Kartuschenkopf 7 lösen.

Die Umlenkeinrichtung 18 hat grob die Form eines Jochs und weist eine Spiegelebene als Symmetrieebene auf, wobei die Achse des Lager- und Mischsystems in der Spiegelebene liegt. Die dem Kartuschenkopf 7 zugewandte Seite der Umlenkeinrichtung 18 besteht aus zwei Zylindersegmenten, die in einer Ebene parallel zu deren Zylinderachse geschnitten sind, wobei an der Mantelfläche der Zylindersegmente die zwei Vertiefungen 37 als Gegenrastungen 37 für ein je ein Rastmittel in den Austragskolben 5, 6 angeordnet sind. Die beiden Zylindersegmente der Umlenkeinrichtung 18 rasten also mit den Austragskolben 5, 6, wenn sie in die hierfür vorgesehenen Öffnungen auf der dem Kartuschenkopf 7 gegenüberliegenden Rückseite der Austragskolben 5, 6 eingesteckt werden.

Die beiden Zylindersegmente sind über eine kreisrunde Platte miteinander verbunden. In der Platte befindet sich der Spalt, der zum Einführen der Trennwand 2 vorgesehen ist, also im eingebauten Zustand mit der Trennwand 2 fluchtet. Auf der Platte erstrecken sich die beiden Wandungen 40, die die geneigte Ablenkwand 22 mit der Ablenkfläche 50 an der Innenseite der Ablenkwand 22. Die Platte, die Wandungen 40 und die Ablenkwand 22 begrenzen die Öffnung 21 und die Durchführung, durch die sich die von der Innenwand der Kartusche 1 gelöste oder abgetrennte Trennwand 2 bewegt, während die Umlenkeinrichtung 18 nach vorne in Richtung des Austragsrohrs 28 bewegt wird. An beiden Seiten des Spalts können sich Schneiden (nicht gezeigt befinden), die die Trennwand 2 im Bereich der Innenwand der Kartusche 1 abtrennen. Erfindungsgemäß bevorzugt befinden sich jedoch keine Schneiden an der Umlenkeinrichtung 18, da die Schneiden den Aufbau unnötig kostenaufwendiger machen würden.

In der Mitte auf der Rückseite der Platte befindet sich ein zentraler senkrechter Zylinder mit einer kreisförmigen Grundfläche 23, die die Anlagefläche 23 für den Stößel 44 der Austragsvorrichtung bildet. Die beiden Zylindersegmente werden über die Platte und die Hülsenwandungen 19 in einem festen Abstand zueinander gehalten. Der Abstand ist so gewählt, dass die beiden Austragskolben 5, 6, wenn sie auf die Zylindersegmente der Umlenkeinrichtung 18 aufgesteckt sind, voneinander mit einem Abstand zueinander gehalten werden, der etwas kleiner oder höchstens genauso groß ist, wie die Stärke der Trennwand 2, also beispielsweise 1 mm. Die Umlenkeinrichtung 18 sollte aus einem Material gefertigt sein, das hart genug ist, um die Trennwand 2 umformen zu können, wenn die Umlenkeinrichtung 18 in der Kartusche 1 in Richtung des Kartuschenkopfs 7 vorgetrieben wird.

Kartusche 1 und Anschluss 10 sind bei allen Varianten bevorzugt einteilig ausgeführt und bestehen bevorzugt aus Kunststoff. Bis auf die Dichtungen 24 können alle Teile der Lagerungs- und Mischsysteme durch Spritzguss aus Kunststoff gefertigt werden. Die Dichtungen 24 bestehen bevorzugt aus Gummi. Auch die Platte 7 für den Kartuschenkopf 7 kann aus Gummi oder einem gummielastischen Werkstoff bestehen. Als Zementkomponenten werden bevorzugt pastöse Ausgangskomponenten eines PMMA-Knochenzements verwendet. Theoretisch können aber auch andere Zemente, wie beispielsweise Dentalzemente, Zweikomponenten-Klebstoffe oder andere Zweikomponenten-Systeme, die aus pastösen Ausgangskomponenten gemischt werden, mit einem erfindungsgemäßen Lagerungs- und Mischsystem gelagert und gemischt werden.

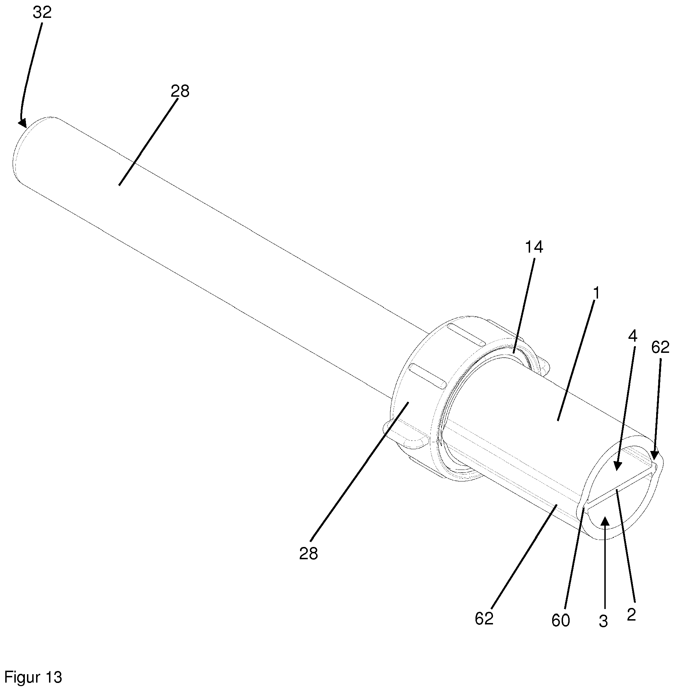

Figur 13 zeigt eine schematische perspektivische Querschnittansicht auf den vorderen Teil eines weiteren alternativen erfindungsgemäßen Lagerungs- und Mischsystems, das mit einem Austragsrohr 28 verbunden ist. Die Trennwand 2 trennt auch hier den Innenraum der Kartusche 1 in zwei Hohlräume 3, 4, die fluiddicht voneinander getrennt sind. Die Trennwand 2 ist nicht einteilig mit der Innenwand der Kartusche 1 verbunden. An dem seitlichen Rand, an dem die Trennwand 2 an der Innenseite der Kartusche 1 anliegt, ist an der Trennwand 2 eine Dichtung 60 vorgesehen, die die Verbindung von der Trennwand 2 zur Innenwand der Kartusche 1 abdichtet. Die Kartusche 1 des Lagerungs- und Mischsystems weist im Innenraum zwei Längsnuten 62 auf, die sich im Innenraum parallel zur Achse der Kartusche 1 erstrecken und in denen die Dichtungen 60 der Trennwand 2 anliegen. In Figur 13 sind die Rückseiten der Längsnuten 62 als Erhebungen zu erkennen und auf einer Seite (in Figur 13 links) als Längsnut 62 bezeichnet. Durch die Längsnuten 62 und die Dichtungen 60 wird eine zur langfristigen Lagerung der Zementkomponenten ausreichende Dichtigkeit der Hohlräume 3, 4 erreicht. Der Aufbau dieser Lagerungs- und Mischeinrichtung entspricht ansonsten den zuvor beschriebenen Lagerungs- und Mischvorrichtungen. Durch die Bewegung der Umlenkeinrichtung 18 wird die Trennwand 2 mit der Dichtung 60 daran von der Innenwand der Kartusche 1 abgehoben. Die Dichtung 60 kann alternativ auch mit der Nut 62 verbunden sein.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartusche
- 2: Trennwand
- 3: Hohlraum
- 4: Hohlraum
- 5: Austragskolben
- 6: Austragskolben
- 7: Kartuschenkopf
- 8: Überwurfmutter
- 9: Stopfen
- 10: Anschluss
- 12: Befestigungselement
- 14: Außengewinde
- 16: Innengewinde
- 18: Umlenkeinrichtung
- 19: Stift / Pilz
- 20: Loch
- 21: Öffnung
- 22: Geneigte Ablenkwand
- 23: Anlagefläche
- 24: Dichtung
- 26: Rastmittel
- 28: Austragsrohr
- 30: Statischer Mischer
- 32: Austragsöffnung
- 34: Innengewinde
- 36: Dichtung
- 37: Gegenrastung / Vertiefung
- 38: Gegenrastmittel / Vertiefung
- 40: Wandung
- 42: Anschluss
- 44: Stößel / Schubstange
- 46: Lagerung
- 50: Geneigte Ablenkfläche
- 58: Rastung
- 60: Dichtung
- 62: Axiale Nut (Rückseite)

## Patentansprüche

1. Lagerungs- und Mischsystem für pastenförmige Zweikomponenten-Polymethylmethacrylat-Knochenzemente, das Lagerungs- und Mischsystem aufweisend
eine röhrenförmige Kartusche (1) mit einem zylindrischen Innenraum,
einen Kartuschenkopf (7), der ein Ende der röhrenförmigen Kartusche (1) an der Vorderseite verschließt,
eine axial in dem zylindrischen Innenraum der Kartusche (1) angeordnete Trennwand (2), wobei die Trennwand (2) den durch den Kartuschenkopf (7) begrenzten zylindrischen Innenraum der Kartusche (1) in zwei räumlich voneinander getrennte Hohlräume (3, 4) teilt, wobei in dem ersten Hohlraum (3) eine erste pastenförmige Zementkomponente enthalten ist und in dem separaten zweiten Hohlraum (4) eine zweite pastenförmige Zementkomponente enthalten ist,
zwei axial in beiden Hohlräumen (3, 4) der Kartusche (1) verschiebbar angeordnete Austragskolben (5, 6), wobei die Austragskolben (5, 6) die beiden Hohlräume (3, 4) auf der dem Kartuschenkopf (7) gegenüberliegenden Rückseite der Hohlräume (3, 4) verschließen,
eine Umlenkeinrichtung (18) zum Umformen der Trennwand (2), wobei die Umlenkeinrichtung (18) in der Kartusche (1) axial bewegbar ist und vom Kartuschenkopf (7) aus gesehen hinter den Austragskolben (5, 6) angeordnet ist oder anzuordnen ist, wobei die Umlenkeinrichtung (18) eine gegen die Achse der Kartusche (1) geneigte Ablenkfläche (50) aufweist, die die Trennwand (2) seitlich in Richtung der Innenwand der Kartusche (1) drückt, wenn die Umlenkeinrichtung (18) in die Kartusche (1) hinein gedrückt wird, wobei
die Trennwand (2) mit der Innenwand der Kartusche (1) über eine Sollbruchstelle verbunden ist oder die Trennwand (2) mit der Innenwand der Kartusche (1) lösbar verbunden ist, so dass sich die Trennwand (2) von der Innenwand der Kartusche (1) löst, wenn die Trennwand (2) durch die Bewegung der Umlenkeinrichtung (18) in die Kartusche (1) hinein verformt wird, wobei
ein rückseitiges Ende der Trennwand (2), das vom Kartuschenkopf (7) aus gesehen hinter den Austragskolben (5, 6) angeordnet ist, an der Kartusche (1) zu befestigen ist oder befestigt ist, so dass sich das rückseitige Ende der Trennwand (2) bei einer Bewegung der Umlenkeinrichtung (18) in die Kartusche (1) hinein nicht von der Kartusche (1) löst.

2. Lagerungs- und Mischsystem nach Anspruch 1, **dadurch gekennzeichnet, dass**
am rückseitigen Ende der Trennwand (2) ein Befestigungselement (20) angeordnet ist, wobei das Befestigungselement (20) an einem Gegenbefestigungselement (19) im Bereich der Rückseite der Kartusche (1) zu befestigen ist, wobei vorzugsweise ein Loch (20) in der Trennwand (2) als Befestigungselement (20) und ein Haken oder ein Stift (19) als Gegenbefestigungselement (19) vorgesehen sind.

3. Lagerungs- und Mischsystem nach einem der vorangehenden Ansprüche, **dadurch gekennzeichnet, dass**
in der Umlenkeinrichtung (18) eine Durchführung angeordnet ist oder die Umlenkeinrichtung (18) mit der Innenwand der Kartusche (1) eine Durchführung bildet, wobei die Ablenkfläche (50) in der Durchführung angeordnet ist und die Trennwand (2) durch die Durchführung zu fädeln ist oder durch die Durchführung gefädelt ist, wobei sich die Trennwand (2) durch die Durchführung bewegt, wenn sich die Umlenkeinrichtung (18) in die Kartusche (1) hinein bewegt.

4. Lagerungs- und Mischsystem nach einem der vorangehenden Ansprüche, **dadurch gekennzeichnet, dass**
die Hohlräume (3, 4) einen halbkreisförmigen oder kreissegmentförmigen Querschnitt aufweisen und die Austragskolben (5, 6) einen dazu passenden Querschnitt aufweisen, so dass die Austragskolben (5, 6) die Hohlräume (3, 4) in jeder axialen Position in den Hohlräumen (3, 4) verschließen.

5. Lagerungs- und Mischsystem nach einem der vorangehenden Ansprüche, **dadurch gekennzeichnet, dass**
die Trennwand (2) an beiden seitlichen Rändern mit jeweils einem Führungselement (62) in oder an der Innenwand der Kartusche (1) lösbar verbunden ist, vorzugsweise in jeweils eine Nut (62) in der Innenwand der Kartusche (1) eingesteckt ist, wobei das Führungselement (62) und die seitlichen Ränder der Trennwand (2) fluiddicht miteinander abschließen.

6. Lagerungs- und Mischsystem nach einem der vorangehenden Ansprüche, **dadurch gekennzeichnet, dass**
die Austragskolben (5, 6) an der dem Kartuschenkopf (7) gegenüberliegenden Rückseite über die Umlenkeinrichtung (18) miteinander verbunden oder verbindbar sind, vorzugsweise die Austragskolben (5, 6) über die Umlenkeinrichtung (18) derart voneinander beabstandet sind, dass der zwischen den Austragskolben (5, 6) liegende Spalt kleiner oder gleich groß ist, wie die Stärke der Trennwand (2).

7. Lagerungs- und Mischsystem nach einem der vorangehenden Ansprüche, **dadurch gekennzeichnet, dass**
in dem Kartuschenkopf (7) zwei Durchführungen vorgesehen sind, die die beiden Hohlräume (3, 4) mit der Umgebung des Lagerungs- und Mischsystems verbinden, wobei in den Durchführungen jeweils ein lösbarer Stopfen (9) angeordnet ist.

8. Lagerungs- und Mischsystem nach einem der vorangehenden Ansprüche, **dadurch gekennzeichnet, dass**
der Kartuschenkopf (7) durch eine gummielastischen Platte (7) realisiert ist, die mit einer Überwurfkappe (8) an der Kartusche (1) befestigt ist, wobei die Überwurfkappe (8) eine Bewegung der gummielastischen Platte (7) von der Kartusche (1) weg mit Hilfe eines überragenden Rands blockiert, und wobei die gummielastische Platte (7) an der den Austragskolben (5, 6) zugewandten Seite eine Aussparung zur Aufnahme der Breitseite der Trennwand (2) besitzt, und wobei vorzugsweise durch diese Aufnahme in der gummielastischen Platte (7) zwei Bereiche definiert sind, wobei in jedem Bereich eine Durchführung angeordnet ist, die durch einen Stopfen (9) verschlossen ist.

9. Lagerungs- und Mischsystem nach einem der vorangehenden Ansprüche, **dadurch gekennzeichnet, dass**
die geneigte Ablenkfläche (50) bereichsweise von einer Wandung (40) der Umlenkeinrichtung (18) umgeben ist, so dass die durch die Umlenkeinrichtung (18) laufende Trennwand (2) von der die geneigte Ablenkfläche (50) umgebenden Wandung (40) der Umlenkeinrichtung (18) um die Längsachse der Trennwand (2) gekrümmt wird.

10. Lagerungs- und Mischsystem nach einem der vorangehenden Ansprüche, **dadurch gekennzeichnet, dass**
die Umlenkeinrichtung (18) als offener Hohlkörper ausgebildet ist, wobei eine Öffnung (21) an der von den Austragskolben (5, 6) abgewandten Rückseite des Hohlkörpers bogenförmig der Innenkontur der Kartusche (1) folgt, wobei die Bogenlänge größer oder gleich der Breite der Trennwand (2) ist.

11. Lagerungs- und Mischsystem nach Anspruch 10, **dadurch gekennzeichnet, dass** eine Verlängerung der Trennwand (2) im Bereich der dem Kartuschenkopf (7) gegenüberliegenden Rückseite der Trennwand (2) durch die rückseitige Öffnung (21) austritt und die Trennwand (2) an diesem Ende an mindestens einem Punkt der Kartusche (1) fixiert ist.

12. Verfahren zur Vermischung von pastenförmigen Zementkomponenten eines pastenförmigen Zementteigs, insbesondere eines Polymethylmethacrylat-Knochenzements, mit einem Lagerungs- und Mischsystem nach mindestens einem der vorangehenden Ansprüche, **gekennzeichnet durch** die folgenden nacheinander ablaufenden Schritte,
a) Entfernen des Kartuschenkopfs (7) von der Kartusche (1) oder Entfernen von zumindest zwei Stopfen (9) aus zumindest zwei Durchführungen im Kartuschenkopf (7), so dass die Kartusche (1) geöffnet wird,
b) Aufsetzen und Verbinden eines Austragsrohrs (28) mit der geöffneten Kartusche (1), wobei das Austragsrohr (28) einen Mischer (30) enthält,
c) Einsetzen der Kartusche (1) in eine manuell zu bedienende Austragsvorrichtung, die Austragsvorrichtung aufweisend einen axial vortreibbaren Stößel (44) zum Vortreiben der Austragskolben (5, 6) in den Hohlräumen (3, 4) der Kartusche (1),
d) Auspressen der pastenförmigen Zementkomponenten mit Hilfe der Austragsvorrichtung durch axiales Vortreiben der Austragskolben (5, 6) mit dem Stößel (44), wodurch die Zementkomponenten in das Austragsrohr (28) gedrückt werden, wobei die beiden Zementkomponenten durch den Mischer (30) im Austragsrohr (28) zu dem pastenförmigen Zementteig gemischt werden, wobei synchron zur Bewegung der Austragskolben (5, 6) die Umlenkeinrichtung (18) über die Trennwand (2) in die Kartusche (1) eingedrückt wird und die Trennwand (2) mit einer Ablenkfläche (50) der Umlenkeinrichtung (18) in Richtung der Innenwand der Kartusche (1) zumindest derart weit in Richtung der Innenwand der Kartusche (1) gedrückt wird, dass eine weitere Bewegung des Stößels (44) der Austragsvorrichtung nicht durch die Trennwand (2) verhindert wird oder nicht durch die Trennwand (2) behindert wird, wobei die Trennwand (2) durch die sich in Richtung des Kartuschenkopfs (7) bewegende Umlenkeinrichtung (18) in axialer Richtung gekrümmt wird und durch die Krümmung der Trennwand (2) die Trennwand (2) von der Innenwand der Kartusche (1) gelöst wird.

13. Verfahren nach Anspruch 12, **dadurch gekennzeichnet, dass**
durch die Krümmung der Trennwand (2) die Trennwand (2) aus Führungselementen (62) herausgelöst wird.

14. Verfahren nach Anspruch 12 oder 13, **dadurch gekennzeichnet, dass**
eine Fixierung die Trennwand (2) an der Rückseite der Kartusche (1) derart fixiert, dass eine axiale Bewegung der Trennwand (2) in den Führungselementen (62) vor den Austragskolben (5, 6) verhindert wird.

15. Verfahren nach einem der Ansprüche 12 bis 14, **dadurch gekennzeichnet, dass**
der Stößel (44) der Austragsvorrichtung auf die von den Austragskolben (5, 6) abgewandte Seite der Umlenkeinrichtung (18) drückt und die Austragskolben (5, 6) über die Umlenkeinrichtung (18) angetrieben werden.

## Claims

1. A storage and mixing system for pasty two-component polymethyl methacrylate bone cements, the storage and mixing system comprising:
a tubular cartridge (1) having a cylindrical inner chamber;
a cartridge head (7), which closes one end of the tubular cartridge (1) on the front side;
a partition (2) disposed axially in the cylindrical inner chamber of the cartridge (1), wherein the partition (2) divides the cylindrical inner chamber of the cartridge (1) bounded by the cartridge head (7) into two cavities (3, 4) that are spatially separated from one another, wherein a first pasty cement component is present in the first cavity (3) and a second pasty cement component is present in the separate second cavity (4);
two dispensing plungers (5, 6) disposed axially displaceably in the two cavities (3, 4) of the cartridge (1), wherein the dispensing plungers (5, 6) close the two cavities (3, 4) on the back side of the cavities (3, 4) situated opposite the cartridge head (7);
a bending device (18) for deforming the partition (2), wherein the bending device (18) is axially movable in the cartridge (1) and is disposed or to be disposed behind the dispensing plungers (5, 6), as seen from the cartridge head (7), wherein the bending device (18) comprises a deflection surface (50) that is inclined with respect to the axis of the cartridge (1) and that presses the partition (2) laterally in the direction of the inside wall of the cartridge (1) when the bending device (18) is pushed into the cartridge (1), wherein
the partition (2) is connected to the inside wall of the cartridge (1) via a predetermined breaking point, or the partition (2) is detachably connected to the inside wall of the cartridge (1), so that the partition (2) detaches from the inside wall of the cartridge (1) when the partition (2) is being deformed by the movement of the bending device (18) into the cartridge (1), wherein
a rear end of the partition (2), which is disposed behind the dispensing plungers (5, 6), as seen from the cartridge head (7), is fixable or is fastened to the cartridge (1) so that the rear end of the partition (2) does not detach from the cartridge (1) when the bending device (18) moves into the cartridge (1).

2. The storage and mixing system according to Claim 1, **characterized in that**
a fastening element (20) is disposed at the rear end of the partition (2), wherein the fastening element (20) is to be fastened to a mating fastening element (19) in the region of the rear side of the cartridge (1), wherein preferably a hole (20) is provided in the partition (2) as the fastening element (20), and a hook or a pin (19) is provided as the mating fastening element (19).

3. The storage and mixing system according to any one of the preceding Claims, **characterized in that**
a passage is provided in the bending device (18), or the bending device (18) forms a passage together with the inside wall of the cartridge (1), wherein the deflection surface (50) is provided in the passage, and the partition (2) is to be fed through the passage or is fed through the passage, wherein the partition (2) moves through the passage when the bending device (18) moves into the cartridge (1).

4. The storage and mixing system according to any one of the preceding Claims, **characterized in that**
the cavities (3, 4) have a semi-circular or circular segment-shaped cross-section, and the dispensing plungers (5, 6) have a matching cross-section, whereby the dispensing plungers (5, 6) close off the cavities (3, 4) in every axial position in the cavities (3, 4).

5. The storage and mixing system according to any one of the preceding Claims, **characterized in that**
the partition (2) is detachably connected on both lateral edges to a respective guide element (62) in or on the inside wall of the cartridge (1), and preferably is inserted into a respective groove (62) in the inside wall of the cartridge (1), wherein the guide element (62) and the lateral edges of the partition (2) are sealed in a fluid-tight manner with one another.

6. The storage and mixing system according to any one of the preceding Claims, **characterized in that**
the dispensing plungers (5, 6) are connected or connectable to one another at the back side situated opposite the cartridge head (7) via the bending device (18), and preferably that the dispensing plungers (5, 6) are disposed at a distance from one another via the bending device (18) such that the gap present between the dispensing plungers (5, 6) is smaller than or equal to the thickness of the partition (2).

7. The storage and mixing system according to any one of the preceding Claims, **characterized in that**
two passages are provided in the cartridge head (7), which connect the two cavities (3, 4) to the surrounding area of the storage and mixing system, wherein a removable plug (9) is disposed in each of the passages.

8. The storage and mixing system according to any one of the preceding Claims, **characterized in that**
the cartridge head (7) is implemented by a rubber-elastic plate (7), which is fastened to the cartridge (1) by way of a safety cap (8), wherein the safety cap (8) blocks a movement of the rubber-elastic plate (7) away from the cartridge (1) with the aid of a protruding edge, and wherein the rubber-elastic plate (7), on the side facing the dispensing plungers (5, 6), has a recess for accommodating the broadside of the partition (2), and wherein two regions are preferably defined by this accommodation in the rubber-elastic plate (7), wherein a passage, which is closed by a plug (9), is provided in each region.

9. The storage and mixing system according to any one of the preceding Claims, **characterized in that**
the inclined deflection surface (50) is surrounded by a wall (40) of the bending device (18) in some regions, so that the partition (2) extending through the bending device (18) is bent about the longitudinal axis of the partition (2) by the wall (40) of the bending device (18) surrounding the inclined deflection surface (50).

10. The storage and mixing system according to any one of the preceding Claims, **characterized in that**
the bending device (18) is designed as an open hollow body, wherein an opening (21) on the back side of the hollow body facing away from the dispensing plungers (5, 6) follows the inner contour of the cartridge (1) in an arc-shaped manner, wherein the length of the arc is greater than or equal to the width of the partition (2).

11. The storage and mixing system according to Claim 10, **characterized in that**
an extension of the partition (2) in the area of the back side of the partition (2) situated opposite the cartridge head (7) exits through the rear opening (21), and the partition (2) is fixed at this end to at least one point of the cartridge (1).

12. A method for mixing pasty cement components of a pasty cement dough, in particular of a polymethyl methacrylate bone cement, using a storage and mixing system according to at least one of the preceding Claims, **characterized by** the following steps taking place consecutively:
a) removing the cartridge head (7) from the cartridge (1), or removing at least two plugs (9) from at least two passages in the cartridge head (7), whereby the cartridge (1) is opened;
b) placing on and connecting a dispensing tube (28) to the opened cartridge (1), wherein the dispensing tube (28) comprises a mixer (30);
c) inserting the cartridge (1) into a manually operable dispensing device, the dispensing device comprising an axially advanceable pusher (44) for advancing the dispensing plungers (5, 6) in the cavities (3, 4) of the cartridge (1);
d) pressing out the pasty cement components with the aid of the dispensing device by axially advancing the dispensing plungers (5, 6) by way of the pusher (44), whereby the cement components are pushed into the dispensing tube (28), wherein the two cement components are mixed by the mixer (30) in the dispensing tube (28) to yield the pasty cement dough, wherein, synchronously with the movement of the dispensing plungers (5, 6), the bending device (18) is pushed over the partition (2) into the cartridge (1), and the partition (2) is pushed by a deflection surface (50) of the bending device (18) in the direction of the inside wall of the cartridge (1) at least so far in the direction of the inside wall of the cartridge (1) that a further movement of the pusher (44) of the dispensing device is not prevented by the partition (2) or is not impaired by the partition (2), wherein the partition (2) is bent in the axial direction by the bending device (18) moving in the direction of the cartridge head (7), and that, as a result of the bending of the partition (2), the partition (2) is detached from the inside wall of the cartridge (1).

13. The method according to Claim 12, **characterized in that**
as a result of the bending of the partition (2), the partition (2) is dislodged from the guide elements (62).

14. The method according to Claim 12 or 13, **characterized in that**
a fixation element fixes the partition (2) to the back side of the cartridge (1) such that an axial movement of the partition (2) in the guide elements (62) in front of the dispensing plungers (5, 6) is prevented.

15. The method according to any one of Claims 12 to 14, **characterized in that**
the pusher (44) of the dispensing device pushes onto the side of the bending device (18) facing away from the dispensing plungers (5, 6), and the dispensing plungers (5, 6) are driven by way of the bending device (18).

## Revendications

1. Système de stockage et de mélange pour ciments osseux à base de polyméthyl méthacrylate bi composant, le système de stockage et de mélange présentant une cartouche en forme de tube (1) dotée d'un espace intérieur cylindrique,
une tête de cartouche (7) qui ferme une extrémité de la cartouche en forme de tube (1) au niveau de la face avant,
une paroi de séparation (2) disposée axialement dans l'espace intérieur cylindrique de la cartouche (1), la paroi de séparation (2) divisant l'espace intérieur cylindrique de la cartouche (1) délimitée par la tête de cartouche (7) en deux cavités (3, 4) séparées spatialement l'une de l'autre, où un premier composant de ciment de forme pâteuse est contenu dans la première cavité (3) et un deuxième composant de ciment de forme pâteuse est contenu dans la seconde cavité (4),
deux pistons d'évacuation (5, 6)) disposés en pouvant coulisser axialement dans les deux cavités (3, 4) de la cartouche (1), où les pistons d'évacuation (5, 6) ferment les deux cavités (3, 4) sur la face arrière des cavités (3, 4) se situant en face de la tête de cartouche (7),
un dispositif de déviation (18) prévu pour déformer la paroi de séparation (2), où le dispositif de déviation (18) est mobile axialement dans la cartouche (1) et est disposé ou est à disposer derrière les pistons d'évacuation (5, 6), vu à partir de la tête de cartouche (7), où le dispositif de déviation (18) présente une surface de déviation (50) inclinée contre l'axe de la cartouche (1) qui presse la paroi de séparation (2) latéralement en direction de la paroi intérieure de la cartouche (1) lorsque que le dispositif de déviation (18) est poussé dans l'intérieur de la cartouche (1), où la paroi de séparation (2) est reliée avec la paroi intérieure de la cartouche (1) par le biais d'un point de rupture souhaité, ou la paroi de séparation (2) est reliée de manière amovible avec la paroi intérieure de la cartouche (1) de sorte que la paroi de séparation (2) se détache de la paroi intérieure de la cartouche (1) lorsque la paroi de séparation (2) est déformée par le déplacement du dispositif de déviation (18) vers l'intérieur de la cartouche (1), où
une extrémité de la face arrière de la paroi de séparation (2), qui est disposée derrière les pistons d'évacuation (5, 6), vu à partir de la tête de cartouche (7), est à fixer ou est fixée sur la cartouche (1), de sorte que l'extrémité de la face arrière de la paroi de séparation (2) ne se détache pas de la cartouche (1) lors d'un déplacement du dispositif de déviation (18) vers l'intérieur de la cartouche.

2. Système de stockage et de mélange selon la revendication 1, **caractérisé en ce qu'** un élément de fixation (20) est disposé à l'extrémité de la face arrière de la paroi de séparation (2), où l'élément de fixation (20) est à fixer sur un élément de fixation complémentaire (19) dans la zone de la face arrière de la cartouche (1), où, de préférence, un trou (20) dans la paroi de séparation (2) est prévu pour servir d'élément de fixation (20) et un crochet ou une tige (19) est prévu pour servir d'élément de fixation complémentaire (19).

3. Système de stockage et de mélange selon l'une des revendications précédentes, **caractérisé en ce**
**qu'**un passage de part en part est agencé dans le dispositif de déviation (18), ou le dispositif de déviation (18) forme un passage de part en part avec la paroi intérieure de la cartouche (1), où la surface de déviation (50) est disposée dans le passage de part en part et la paroi de séparation (2) doit être enfilée à travers le passage de part en part ou est enfilée à travers le passage de part en part, où la paroi de séparation (2) se déplace à travers le passage de part en part lorsque le dispositif de déviation (18) se déplace vers l'intérieur de la cartouche (1).

4. Système de stockage et de mélange selon l'une des revendications précédentes, **caractérisé en ce que**
les cavités (3, 4) présentent une section transversale en forme de demi cercle ou en forme de segment de cercle et les pistons d'évacuation (5, 6) présentent une section transversale y étant assortie de sorte que les pistons d'évacuation (5, 6) ferment les cavités (3, 4) dans chaque position axiale dans les cavités (3, 4).

5. Système de stockage et de mélange selon l'une des revendications précédentes, **caractérisé en ce que**
la paroi de séparation (2) est reliée de manière amovible au niveau de deux roues latérales avec respectivement un élément de guidage (62) dans ou sur la paroi intérieure de la cartouche (1), de préférence dans respectivement une rainure (62) dans la paroi intérieure de la cartouche (2), où l'élément de guidage (62) et les roues latérales de la paroi de séparation (2) forment ensemble une fermeture hermétique par rapport aux fluides.

6. Système de stockage et de mélange selon l'une des revendications précédentes, **caractérisé en ce que**
les pistons d'évacuations (5, 6) sont reliés ou peuvent être reliés ensemble sur la face arrière située en face de la tête de cartouche (7) par le biais du dispositif de déviation (18), de préférence, les pistons d'évacuation (5, 6) sont espacés l'un de l'autre par le biais du dispositif de déviation (18) de telle manière que la fente située entre les pistons d'évacuation (5, 6) est plus petite ou est de la même taille que l'épaisseur de la paroi de séparation (2).

7. Système de stockage et de mélange selon l'une des revendications précédentes, **caractérisé en ce que**
deux passages de part en part sont prévus dans la tête de cartouche (7) qui relient les deux cavités (3, 4) avec l'environnement du système de stockage et de mélange, où respectivement un bouchon (9) amovible est disposé dans les passages de part en part.

8. Système de stockage et de mélange selon l'une des revendications précédentes, **caractérisé en ce que**
la tête de cartouche (7) est réalisée par une plaque (7) en caoutchouc élastique qui est fixée sur la cartouche (1) avec un bouchon à vis (8), où le bouchon à vis (8) bloque un déplacement d'éloignement à partir de la cartouche (1) de la plaque (7) en caoutchouc élastique à l'aide d'un rebord protubérant, et où la plaque (7) en caoutchouc élastique possède un renfoncement sur le côté orienté vers le piston d'évacuation (5, 6) pour la réception du côté large de la paroi de séparation (2), et où de préférence deux zones sont définies par cette réception dans la plaque (7) en caoutchouc élastique, où un passage de part en part est disposé dans chaque zone qui est fermé par un bouchon (9).

9. Système de stockage et de mélange selon l'une des revendications précédentes, **caractérisé en ce que**
la surface de déviation (50) inclinée est entourée par endroits par une paroi (40) du dispositif de déviation (18) de sorte que la paroi de séparation (2) s'étendant à travers le dispositif de déviation (18) est recourbée autour de l'axe longitudinal de la paroi de séparation (2) par la paroi (40) entourant la surface de déviation (50) inclinée.

10. Système de stockage et de mélange selon l'une des revendications précédentes, **caractérisé en ce que**
le dispositif de déviation (18) est conçu sous la forme d'une cavité ouverte, où une ouverture (21) sur la face arrière de la cavité opposée au piston d'évacuation (5, 6) suit le contour intérieur de la cartouche (1) en forme d'un arc, où la longueur de l'arc est supérieure ou égale à la largeur de la paroi de séparation (2).

11. Système de stockage et de mélange selon la revendication 10, **caractérisé en ce qu'**un prolongement de la paroi de séparation (2) sort par l'ouverture (21) du côté arrière dans la zone du côté arrière de la paroi de séparation (2) situé en face de la tête de cartouche (7) et la paroi de séparation (2) est fixée à cette extrémité au niveau d'au moins un point de la cartouche (1).

12. Procédé de mélange de composants de ciment sous forme pâteuse d'une pâte de ciment en forme pâteuse, notamment d'un ciment osseux à base de polyméthyl méthacrylate, avec un système de stockage et de mélange selon au moins une des revendications précédentes, **caractérisé par** les étapes suivantes se déroulant l'une après l'autre,
a) le retrait de la tête de cartouche (7) de la cartouche (1) ou le retrait d'au moins deux bouchons (9) à partir d'au moins deux passages de part en part dans la tête de cartouche (7) de sorte que la cartouche (1) est ouverte,
b) le montage et la mise en communication d'un tube d'évacuation (28) avec la cartouche (1) ouverte, où le tube d'évacuation (28) contient un mélangeur (30),
c) l'insertion de la cartouche (1) dans un dispositif d'évacuation à actionner manuellement, le dispositif d'évacuation présentant un poussoir (44) à actionner axialement pour pousser le piston d'évacuation (5, 6) vers l'avant dans les cavités (3, 4) de la cartouche (1),
d) le pressage des composants de ciment de forme pâteuse à l'aide du dispositif d'évacuation par un avancement axial des pistons d'évacuation (5, 6) avec le poussoir (44), ce par quoi les composants de ciment sont pressés dans le tube d'évacuation (28), où les deux composants de ciment sont mélangés par le mélangeur (30) dans le tube d'évacuation (28) en une pâte de ciment de forme pâteuse, où le dispositif de déviation (18) est pressé dans la cartouche (1) de manière synchronisée au déplacement des pistons d'évacuation (5, 6) par le biais de la paroi de séparation (2), et la paroi de séparation (2) est pressée avec une surface de déviation (50) du dispositif de déviation (18) en direction de la paroi intérieure de la cartouche (1) au moins aussi loin en direction de la paroi intérieure de la cartouche (1) qu'un nouveau déplacement du poussoir (44) du dispositif d'évacuation n'est pas empêché par la paroi de séparation (2) ou n'est pas gêné par la paroi de séparation (2), où la paroi de séparation (2) est recourbée en direction axiale par le dispositif de déviation (18) se déplaçant en direction de la tête de cartouche (7) et la paroi de séparation (2) est détachée de la paroi intérieure de la cartouche (1) par la courbure de la paroi de séparation (2).

13. Procédé selon la revendication 12, **caractérisé en ce que**
la paroi de séparation (2) est détachée des éléments de guidage (62) par la courbure de la paroi de séparation (2).

14. Procédé selon la revendication 12 ou la revendication 14, **caractérisé en ce**
**qu'**une fixation de la paroi de séparation (2) sur le côté arrière de la cartouche (1) est efficace de telle manière qu'un déplacement axial de la paroi de séparation (2) dans les éléments de guidage (62) est empêché avant les pistons d'évacuation (5, 6).

15. Procédé selon l'une des revendications 12 à 14, **caractérisé en ce que**
le poussoir (44) du dispositif d'évacuation presse sur le côté du dispositif de déviation (18) opposé aux pistons d'évacuation (5, 6) et les pistons d'évacuation (5, 6) sont entraînés par le biais du dispositif de déviation (18).
